# EUROPEAN PATENT SPECIFICATION

(11) **EP 1 416 016 B1**
(45) Date of publication and mention of the grant of the patent: **13.08.2008**
(21) Application number: 03078459.9
(22) Date of filing: 03.11.2003
(51) Int. Cl.: C08L 83/04, C08K 3/00

(54) **Powder composition, a dispersion of powder in oil, and a cosmetic preparation comprising polyglycerine modified silicone**
Pulverdispersion, Dispersion von Pulver in Öl und kosmetische Präparation enhaltend polyglycerinmodifiziertes Silikon
Composition pulverulente, dispersion de ladite composition dans de l'huile et preparation cosmetique contenant d'un silicone modifié de polyglycerine

(30) Priority: 01.11.2002 JP 2002320478
(43) Date of publication of application: 06.05.2004
(73) Proprietor: Shin-Etsu Chemical Co., Ltd., Chiyoda-ku, Tokyo 100-0004 (JP)
(72) Inventor: Kamei, Masanao Silicone-Electronic Mat. Res. Ctr., Matsuida-machi Usui-gun Gunma 379-0222 (JP); Tachibana, Kiyomi, Chiyoda-ku Tokyo 100-0004 (JP)
(74) Representative: Prins, Adrianus Willem

(56) References cited:
- EP-A- 1 213 316
- EP-A- 1 327 668
- WO-A-02/08336
- PATENT ABSTRACTS OF JAPAN vol. 1999, no. 03, 31 March 1999 (1999-03-31) & JP 10 316536 A (KOSE CORP), 2 December 1998 (1998-12-02)

## Description

### FIELD OF THE INVENTION

The present invention relates to a dispersion of powder in oil, consisting of a polyglycerin-modified silicone having at least one silicone branch, powder and/or a coloring agent, and an oily medium

### PRIOR ART

Generally, secretions such as sweat, tears and sebum cause makeup runs. Especially, in suncut agents and makeup cosmetics, together with an oil agent contained in cosmetics, sebum secreted from skin excessively wets powder in cosmetics, which is a main cause for makeup runs. In order to reduce the amount of a cosmetic oil remaining on the skin, an attempt was made to use a volatile oil such as octamethylcyclotetrasiloxane or decamethylcyclopentasiloxane as a part of oil ingredients to be added.

Rubbing and water are also an external factor causing poor coverage of cosmetics. In order to improve such poor makeup coverage caused by substances which are soluble in sweat or water, or in order to prevent loss of water-soluble components or sebum from skin and to maintain a protective effect of skin, silicone oil has been added to enhance water repellency. Since silicone oils such as dimethylpolysiloxane have superior features such as light touch, excellent water repellency, and high safety when applied, they have been often used as an oil agent in cosmetics.

On the other hand, pigments such as such as titanium dioxide, zinc oxide, and red iron oxide, and powder such as mica and serisite, are widely used in the field of cosmetics, such as suncuts, nail colors, nail coats, foundations, mascaras, and eye liners. This kind of powder is usually treated with alumina, silica, oil, or metallic soap, or surface treated with organopolysiloxane to block surface activity or to provide water resistance, sebum resistance, and dispersibility.

Especially these years, use is often made of organopolysiloxane which has a reactive site in the molecule. This is effective to improve surface properties of powder and to block surface activity since this forms chemical bonds with powder surface. Other advantages of the treatment are that the treatment is performed without failure and that the treatment is efficient since the agent does not leave off the powder surface when it is applied in solvent-borne cosmetics and change in the properties due to the treatment is minor.

For instance, Japanese Patent No. 2719303 discloses a process for surface treatment, wherein 12 to 60 parts by weight of methylhydrogenpolysiloxane, relative to 100 parts by weight of powder, are used.

Japanese Laid-Open Patent Application No. 7-196946 discloses a process for surface treatment, wherein linear silicone modified with an alkoxy group at one end is used. As described above, powder treatment with reactive organopolysiloxane is generally known, but none of the prior powder treatment processes are satisfactory since there still remains such a problem that, especially in the case of a methylhydrogenpolysiloxane type of agents, such as methylhydrogenpolysiloxane and dimethylmethylhydrogenpolysiloxane, unreacted Si-H remains after the agent is applied in the surface treatment, and then a hydrogen gas may arise when the so-treated powder is blended in a cosmetic, depending on the condition of the cosmetic.

The powder surface-treated with silicone modified with an alkoxyl group at one end is less problematic in water resistance and sebum resistance when it is used in pressed powder cosmetics, while the treatment effect is not satisfactory when used in solvent-borne cosmetics. The reason for this may be that the silicone modified with an alkoxy group at one end has less reactive sites than a methylhydrogenpolysiloxane type of agents and, therefore, to powder surface remains untreated more when after treated with the former. When the powder thus treated is dispersed in oil, dispersibility is improved, but not satisfactory.

Japanese Laid-Open Patent Application No. 10-316536 discloses a modified powder which is treated with polyglycerin-modified silicone. However, the silicone has a linear structure and has insufficient redispersibility in oil to cause separation of a dispersion with time and poor re-dispersibility, and may sometimes lower the quality of products or user's satisfaction.

The silicone is this application does not comprise the organosilicone groups R³ of formula 5 in the present application.

Japanese Laid-Open Patent Application No. 2002-38013 discloses a powder composition treated with modified silicone having an alcoholic OH group, such as triglycerol derivatives. There, triglycerol whose hydroxyl groups are protected with acetal is subjected to substitution reaction and then to de-acetone reaction, which results in a prolonged preparation process and a reduced pot yield. Further, if the de-acetone reaction is insufficient and the resulting silicone is used in a cosmetic, acetone may arise with time to give unpleasant odor.

EP-A-1 213 316 describes emulsified cosmetics containing polyglycerin modified silicone and powder. However, this document teaches that the silicone is an emulsifier to bridge between an oily component and an aqueous component. It does not disclose powder dispersion.

### SUMMARY OF THE INVENTION

The object of the invention is to provide a powder composition having blocked surface activity, good water resistance, sebum resistance, less tendency to aggregation, and an excellent dispersibility in various oils and also to provide a dispersion thereof in oil and a cosmetic comprising the same which has a good stability with time and can meet user's satisfaction.

The inventors have made intensive studies in order to achieve the above object of the present invention and have found that the object can be achieved by a powder composition comprising polyglycerin-modified silicone having at least one silicone branch and powder and/or a coloring agent, a dispersion of powder in oil comprising such silicone, powder and/or a coloring agent, and an oily medium, and cosmetics comprising the same.

The inventors have found that polyglycerin-modified silicone having at least one silicone branch according to the present invention has stable dispersibility in the oily medium since it has branched structure. Further, the alcoholic OH groups of the silicone are localized, so that the silicone has good absorption toward the powder and/or a coloring agent and does not separate from powder and/or a coloring agent. Thus the dispersion is stable with time.

The inventors have found that especially when powder is surface treated with the polyglycerin-modified silicone having at least one silicone branch as a powder treatment agent, the surface activity of the powder can be blocked. When this is used in cosmetics, and this gives a dry touch to the cosmetics, and long lasting makeup coverage on account of the good water resistance and good sebum resistance. Thus the inventors have completed the invention.

### DESCRIPTION OF THE PREFERRED EMBODIMENTS

The polyglycerin-modified silicone compound having at least one silicone branch which is used in the present invention has the follwing formula (1).

R¹ₐR²_{b}R³_{c}SiO_{(4-a-b-c)/ 2} (1)

Examples of R¹ include alkyl groups such as methyl, ethyl, propyl, butyl, pentyl, hexyl, heptyl, octyl, nonyl, and decyl; cycloalkyl groups such as cyclopentyl and cyclohexyl; aryl groups such as phenyl and tolyl; aralkyl groups such as benzyl and phenetyl; fluorinated alkyl groups such as trifluoropropyl and heptadecafluorodecyl; amino-substituted alkyl groups such as 3-aminopropyl, 3-[(2-aminoethyl)amino]propyl; and carboxyl-substituted alkyl groups such as 3-carboxyproply.

Some of R¹ may be an organic group represented by the following general formula (2), -C_{d}H_{2d} - O - (C₂H₄O)ₑ (C₃H₆O)_{f}R⁴. R⁴ is an organic group, C₁₋₃₀ hydrocarbyl group or R⁵-(CO)-, wherein R⁵ is a C₁₋₃₀ hydrocarbyl group.

Particularly, preferably at least 50 %, more preferably at least 70 %, of the whole R¹ is methyl. Even 100 % of the whole R¹ can be methyl.

The formula (2) represents a residue of alcohol or residue of adduct of alkenylether and d, e, and f are integers of 0 ≤ d ≤ 15, 0 ≤ e ≤ 50, and 0 ≤ f ≤ 50.

Specifically, when d = 0, the formula (2) is -O- (C₂H₄O)ₑ(C₃H₆O)_{f}R⁴. If d = 0, e = 0 and f= 0, the formula (2) represents a C₁₋₃₀ alkoxy group such as a lower alkoxy group such as methoxy and butoxy or a higher alkoxy group such as cetyloxy group, oleyloxy group, and stearloxy derived from cetyl alcohol, oleyl alcohol, and stearyl alcohol, respectively, or a residue of carboxylic acid such as acetic acid, lactic acid, butyric acid, oleic acid, stearic acid, and behenyl acid. If d = 0, e > 1 and f > 1, it represents an alcoholic residue of alkylene oxide adduct of higher alcohol with a terminal hydroxyl group. If d ≥ 1, e = 0, and f = 0, d is preferably 3, 5, or 11 and then the formula (2) represents a residue of propyl ether, pentyl ether, or undecyl ether. More particularly, the formula (2) may be a residue of propyl stearyl ether, pentyl behenyl ether, or undecyl oleyl ether, depending on what R⁴ is.

If d ≥ 1 and e or f is not 0, an alkoxy or ester group is present, bonded via a polyoxyalkylene group in the residue presented by the formula (2). Preferably, d is between 3 and 5 regardless of the values of e and f. If d is 0, the organopolysiloxane is less resistant to hydrolysis and if d is 15 or more, the organosiloxane has a strong oily odor.

R² has the structure represented by formua (3) or (4), wherein R⁴ is a hydrogen atom, a C₁₋₃₀ alkyl group, or an organic group having the formula, R⁵-(CO)-, R⁵ being a C₁. ₃₀ hydrocarbyl group.

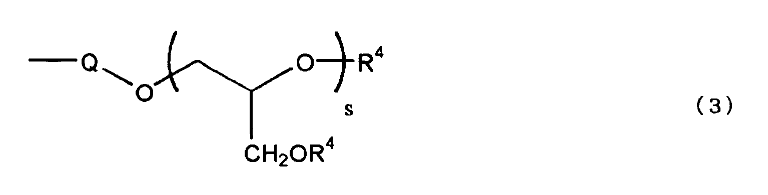

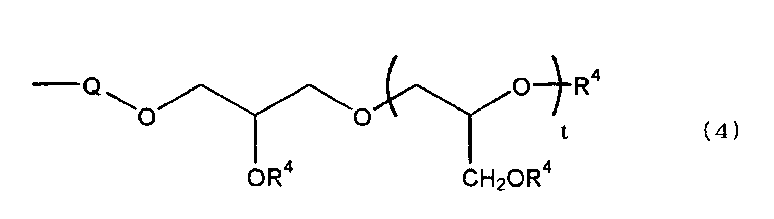

Q is a divalent C_{3 - 20} hydrocarbyl group which may contain an ether or ester bond. Examples of Q include -(CH₂)₂-, -(CH₂)₃-, -CH₂CH(CH₃)CH₂-, -(CH₂)₄-, -(CH₂)₅-, -(CH₂)₆-, -(CH₂)₇-, -(CH₂)₈-, -(CH₂)₂-CH(CH₂CH₂CH₃)-, -CH₂-CH(CH₂CH₃)-, -(CH₂)₃-O-(CH₂)₂-, -(CH₂)₃-O-(CH₂)₂-O-(CH₂)₂-, -(CH₂)₃-O-CH₂CH(CH₃)-, -CH₂-CH(CH₃)-COO(CH₂)₂-, wherein s is an integer of 2 to 20, and t is an integer of 1 to 20.

R³ is an organopolysilicone group having the general formula (5), 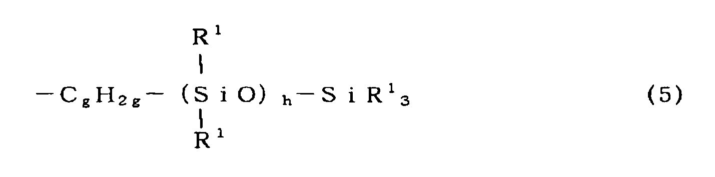 wherein, g is an integer in the range of 1 to 5. Especially when the organopolysiloxyl group is introduced via a reaction of vinylsiloxy group and a SiH group, g is 2. The letter, h, is an integer in the range of 0 to 500, preferably, of 1 to 50. If h is larger than 500, a problem may occur that its reactivity with a main chain is lower.

The above-described organopolysiloxyl group (5) can be introduced from siloxane with a vinyl group at one end which is prepared in an equilibration reaction between divinyltetramethyldisiloxane and hexamethyldisiloxane or octamethylcyclotetrasiloxane according to known methods or from a vinylsiloxane compound with a higher single terminal blocking ratio, prepared from ring-opening polymerization of hexamethylcyclotrisiloxane using a pentaligand silicon complex catalyst or an anionic polymerization catalyst.

The silicone compound (1) according to the present invention may be easily prepared by addition reaction between organohydrogenpolysiloxane and, for instance, an allyl ether compound according to the following formula (i) or (ii), vinyl silicone compound according to the following formula (iii), or an alkylene compound such as hexene in the presence of platinum catalyst or rhodium catalyst.

CH₂=CHCH₂-O- (C₂H₄O)ₑ(C₃H₆O)_{f}-R⁴ (i)

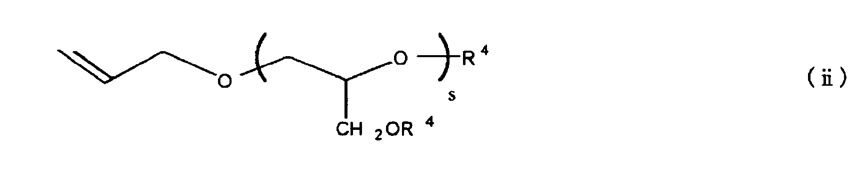

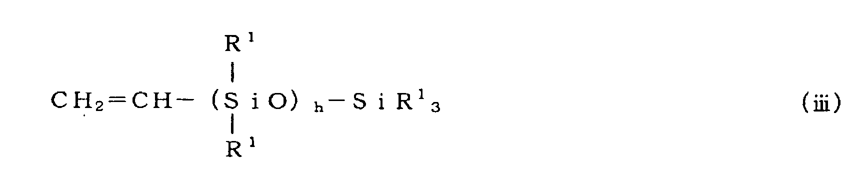

wherein R¹, R⁴, e, f, h, and s have the same meaning as defined above. The organohydrogenpolysiloxane here may be either linear or cyclic. For smoother progress of the addition reaction, linear one is advantageous.

The mixing ratio between the organohydrogenpolysiloxane and the total of the polyglycerin compounds according to the above general formula (ii), the silicone compound according to the above general formula (iii), the alkylene compound and the organic compound according to the above general formula (i) is such as to give a molar ratio of the SiH to the terminal unsaturated groups of 0.5 to 2.0, preferably 0.8 to 1.2.

It is desirable to carry out the above-described addition reaction in the presence of platinum catalyst or rhodium catalyst, preferably chloroplatinic acid, alcohol-modified chloroplatinic acid and chloroplatinic acid-vinylsiloxane complex.

The catalyst can be used in a conventional catalytic amount, preferably in 50 ppm or less, more preferably 20 ppm or less, of the amount of platinum or rhodium. The above addition reaction may be carried out in an organic solvent as required. Examples of the organic solvent include aliphatic alcohols such as methanol, ethanol, 2-propanol and butanol; aromatic hydrocarbons such as toluene and xylene; aliphatic or alicyclic hydrocarbons such as n-pentane, n-hexane and cyclohexane; and halogenated hydrocarbons such as dichloromethane, chloroform and carbon tetrachloride. The conditions of addition reaction are not particular limited, but reaction is preferably performed for 1 to 10 hours under reflux.

In the formula (1), "a" is from 1.0 to 2.5, preferably from 1.2 to 2.3. If "a" is smaller than 1.0, the compatibility with the oily medium is too low to obtain water resistance. If "a" is larger than 2.5, the hydrophilicity is lower so that the reactivity with powder is too low to obtain a stable dispersion. The preferred range of b is from 0.001 to 1.5, more preferably from 0.05 to 1.0. If b is smaller than 0.001, the hydrophilicity is lower so that the reactivity with powder is too low to obtain a stable dispersion. If b is greater than 1.5, the hydrophilicity is too high to obtain a stable dispersion. The preferred range of c is from 0.001 to 1.5, preferably from 0.05 to 1.0. If c is smaller than 0.001, the compatibility with silicone oils is too low to obtain a stable dispersion. If c is greater than 1.5, the hydrophilicity is lower so that the reactivity with powder is too low to obtain a stable dispersion.

Weight average molecular weight of the present silicone compound of the formula (1) is not particularly limited, but is preferably in the range of 500 to 200,000, particularly 1,000 to 100,000. If the weight average molecular weight is greater than 100,000, its viscosity is so high that cosmetics formulated with the powder whose surface has been treated with the organosilicone compound fails to obtain a good user's satisfaction. On the other hand, if the weight average molecular weight is 300 or less, smoothness characteristic of silicone is not obtained. Particularly, the weight average molecular weight is in the range of 1,000 to 10,000.

The powder and/or the coloring agent that may be used in the dispersion of powder in oil according to the present invention may be any powder which is commonly used in cosmetics, regardless of its shape such as spherical, needle or plate, its particle diameter such as fume, fine particle, or pigment grade, and its particle structure such as porous or non-porous. Examples of the powder or the coloring agent include inorganic powder, organic powder, powder of metal salts of surfactants, colored pigments, pearl pigments, metallic powder pigments, and natural colors.

Specific examples of the inorganic powder include titanium dioxide, zirconium oxide, zinc oxide, cerium oxide, magnesium oxide, barium sulfate, calcium sulfate, magnesium sulfate, calcium carbonate, magnesium carbonate, talc, mica, kaolin, sericite, white mica, synthetic mica, phlogopite, lepidolite, biotite, lithia mica, silicic acid, silicic anhydride, aluminum silicate, magnesium silicate, aluminum magnesium silicate, calcium silicate, barium silicate, strontium silicate, metal salts of tungstenic acid, hydroxyapatite, vermiculite, higilite, bentonite, montmorillonite, hectolitre, zeolite, ceramics powder, calcium secondary phosphate, alumina, aluminum hydroxide, boron nitride, and silica.

Examples of the organic powder include polyamide powder, polyester powder, polyethylene powder, polypropylene powder, polystyrene powder, polyurethane powder, benzoguanamine powder, polymethylbenzoguanamine powder, tetrafluoroethylene powder, polymethylmethacrylate powder, cellulose powder, silk powder, powder of nylon such as Nylon 12 and Nylon 6, fine powder of crosslinked silicone with crosslinked dimethylsilicone structure, block copolymers of crosslinked silicone and network structure silicone, fine powder of polymethylsesquioxane, powder of styrene / acrylic acid copolymer, divinylbenzene / styrene copolymer, vinyl resin, urea resin, phenol resin, fluororesin, silicone resin, acrylic resin, melamine resin, epoxy resin, polycarbonate resin, microcrystalline fiber, starch powder, and lauroyl lysine.

Examples of the powder of metal salts of surfactants, i.e. metal soaps, include zinc stearate, aluminum stearate, calcium stearate, magnesium stearate, zinc myristate, magnesium myristate, zinc cetyl phosphate, calcium cetyl phosphate, and zinc sodium cetyl phosphate. Examples of the colored pigments include inorganic red pigments such as iron oxide, iron hydroxide, and iron titanate, inorganic brown pigments such as γ⁻ iron oxide, inorganic yellow pigments such as iron oxide yellow and loess, inorganic black pigments such as iron oxide black and carbon black, inorganic violet pigments such as manganese violet and cobalt violet, inorganic green pigments such as chromium hydroxide, chromium oxide, cobalt oxide, and cobalt titanate, inorganic blue pigments such as Prussian blue and ultramarine blue, lakes of tar pigments, lakes of natural dyes, and synthetic resin powder, composite thereof.

Examples of the pearl pigments include titanium dioxide-coated mica, bismuth oxychloride, titanium dioxide-coated bismuth oxychloride, titanium dioxide-coated talc, fish scales, and titanium dioxide-coated colored mica; metallic powder pigments such as aluminum powder, copper powder and stainless steel powder; tar pigments such as Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 505, Yellow No. 4, Yellow No. 5, Yellow No. 202, Yellow No. 203, Yellow No. 204, Yellow No. 401, Blue No. 1, Blue No. 2, Blue No. 201, Blue No. 404, Green No. 3, Green No. 201, Green No. 204, Green No. 205, Orange No. 201, Orange No. 203, Orange No. 204, Orange No. 206, and Orange No. 207; and natural pigments such as carminic acid, laccaic acid, carthamin, brazilin, and crocin.

When the surface of these powders is treated with the polyglycerin-modified silicone according to the formula (1), the polyglycerin-modified silicone is preferably added in an amount of 0.1 to 30 parts by weight, more preferably 0.5 to 10 parts by weight, relative to 100 parts by weight of the powder and/or the coloring agent.

The polyglycerin-modified silicone having at least one silicone branch according to the formula (1) can be applied on the surface of powder in accordance with known methods. For instance, s suitable method may be selected from the following:
1. a method where the surface is treated by dispersing the objective powder in an organic solvent medium containing a treatment agent,
2. a method where the surface is treated by mixing powder with a treatment agent, followed by treatment in a mill such as a ball mill and a jet mill, and
3. a method where the surface is treated by dispersing powder in a solvent containing a treatment agent, so that the powder absorbs the agent on the surface, and then dried and baked.

Any liquid oil which is commonly used in cosmetics may be used as the oily medium that is used in a dispersion of powder in oil according to the present invention.

Examples of the silicone oils which can be used as the oily medium include organopolysiloxanes having low or high viscosity such as dimethylpolysiloxane, methylphenylpolysiloxane, methylhydrogenpolysiloxane and dimethylsiloxane-methylphenylsiloxane copolymer; cyclosiloxanes, such as octamethylcyclotetrasiloxane, decamethylcyclopentasiloxane, dodecamethylcyclohexasiloxane, tetramethyltetrahydrogencyclotetrasiloxane, tetramethyltetraphenylcyclotetrasiloxane, tetramethyltetratrifluoropropyl, and cyclotetrasiloxane, pentamethylpentatrifluoropropyl cyclopentasiloxane; solution in cyclosiloxane of silicone rubbers, such as gummy dimethylpolysiloxanes and gummy dimethylsiloxane-methylphenylsiloxane copolymers having a high polymerization degree; trimethylsiloxysilicate, solution in cyclosiloxane of trimethylsiloxysilicate, alkyl-modified silicones, amino-modified silicones, fluorine-modified silicones, silicone resin and silicone resin solutions. Fluorine-containing oils may also be used, such as perfluoropolyether, perfluorodecalin and perfluorooctane.

Examples of the hydrocarbon oils which can be used as the oily medium include linear, branched, and volatile hydrocarbon oils, such as α-olefin oligomers, light isoparaffin, light liquid isoparaffin, squalane, synthetic squalane, plant squalane, liquid paraffin, and liquid isoparaffin.

Examples of the ester oils which can be used as the oily medium include diisobutyl adipate, 2-hexyldecyl adipate, di-2-heptylundecyl adipate, N-alkyl glycol monoisostearate, isocetyl isostearate, trimethylolpropane triisostearate, isononyl isononanate, isotridecyl isononanate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, neopentyl glycol dicaprirate, triethyl citrate, 2-ethylhexyl succinate, amyl acetate, ethyl acetate, butyl acetate, isocetyl stearate, butyl stearate, diisopropyl sebacinate, di-2-ethylhexyl sebacinate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerythritol fatty acid esters, isopropyl myristate, octyldodecyl myristate, 2-hexyldecyl myristate, myristyl myristate, hexyldecyl dimethyloctanoate, ethyl laurate, hexyl laurate, 2-octyldodecyl N-lauroyl-L-glutamate, and diisostearyl malate.

Examples of the glyceride oils which can be used as the oily medium include acetoglyceryl, glycerol triisooctanoate, glyceryl triisostearate, glyceryl triisopalmitate, glyceryl monostearate, glyceryl di-2-heptylundecanoate, glyceryl trimyristate, and diglyceryl myristyl isostearate.

Examples of the higher fatty acids which can be used as the oily medium include undecylenic acid, oleic acid, linoleic acid, linolenic acid, arachidonic acid, eicosapentaenoic acid (EPA), docosahexaenoic acid (DHA), isostearic acid, lactic acid. Higher alcohols may also be used, such as oleoyl alcohol, isostearyl alcohol, hexyldecanol, octyldodecanol, cetostearyl alcohol, 2-decyltetradecinol, and monooleyl glyceryl ether (cerakyl alcohol).

Examples of the animal or plant oils and semisynthetic oils which can be used as the oily medium are as follows: avocado oil, almond oil, olive oil, liver oil, neat's-foot oil, apricot kernel oil, wheat germ oil, sesame oil, rice germ oil, rice bran oil, camellia kissi seed oil, safflower oil, cinnamon oil, turtle oil, soybean oil, tea seed oil, camellia oil, evening primrose oil, corn oil, rapeseed oil, Japanese tung oil, germ oil, persic oil, castor oil, castor oil fatty acid methyl ester, sunflower oil, grape oil, jojoba oil, macadamia nut oil, mink oil, meadowfoam oil cottonseed oil, tri-coconut oil fatty acid glyceride, peanut oil, liquid lanolin, lanolin acetate, POE lanolin alcohol ether, and egg yolk oil.

The dispersion of powder in oil can be easily prepared according to any known processes, for instance,
1. a method where the powder composition obtained in accordance with the above-described processes is added into oil such as an ester oil or a silicone oil to disperse, or
2. a method where a silicone compound is dissolved or dispersed in the above-described oil, to which powder is added, and mixed by a mill such as ball mill, beads mill or sand mill.
The resulting dispersion of powder in oil can be blended as such in cosmetic.

The dispersion of powder in oil according to the present invention can be applied for various use and are particularly suitable as a raw material for all kinds of cosmetics that are applied on skin or hair such as skin care products, makeup products, hair care products, antiperspirant products, and UV-ray protection products. Depending on kind and form of cosmetics, 0.1 to 99 wt.%, based on the total amount of the cosmetics, of the powder composition (A) dispersion of powder in oil may be added to the cosmetics.

Depending on the aim of the cosmetic according to the present invention, the cosmetic can contain one or more unctuous agents (B), which is commonly used for cosmetics and may be solid, semisolid, or liquid.

Examples of the natural animal or plant oils and semisynthetic oils which can be used as (B) include avocado oil, linseed oil, almond oil, Ibota wax, perilla oil, olive oil, cacao butter, kapok wax, kaya oil, carnauba wax, liver oil, candelilla wax, beef tallow, neat's-foot oil, beef bone fat , hydrogenated beef tallow, apricot kernel oil, spermaceti wax, hydrogenated oil, wheat germ oil, sesame oil, rice germ oil, rice bran oil, sugar cane wax, camellia kissi seed oil, safflower oil, shear butter, Chinese tung oil, cinnamon oil, jojoba wax, shellac wax, turtle oil, soybean oil, tea seed oil, camellia oil, evening primrose oil, corn oil, lard, rapeseed oil, Japanese tung oil, rice bran oil, germ oil, horse fat, persic oil, palm oil, palm kernel oil, castor oil, hydrogenated castor oil, castor oil fatty acid methyl ester, sunflower oil, grape oil, bayberry wax, jojoba oil, macadamia nut oil, beeswax, mink oil, cottonseed oil, cotton wax, Japanese wax, Japanese wax kernel oil, montan wax, coconut oil, hydrogenated coconut oil, tri-coconut oil fatty acid glyceride, mutton tallow, peanut oil, lanolin, liquid lanolin, hydrogenated lanolin, lanolin alcohol, hard lanolin, lanolin acetate, isopropyl lanolate, hexyl laurate, POE lanolin alcohol ether, POE lanolin alcohol acetate, polyethylene glycol lanolate, POE hydrogenated lanolin alcohol ether, and egg yolk oil, wherein POE means polyoxyethylene.

Examples of the hydrocarbon oils which can be used as (B) include ozokerite, squalane, squalene, ceresin, paraffin, paraffin wax, liquid paraffin, pristane, polyisobutylene, microcrystalline wax, vaseline and higher fatty acids, e.g., lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid (DHA), isostearic acid, and 12-hydroxystearic acid.

Examples of the higher alcohols which can be used as (B) include lauryl alcohol, myristyl alcohol, palmityl alcohol, stearyl alcohol, behenyl alcohol, hexadecyl alcohol, oleyl alcohol, isostearyl alcohol, hexyldodecanol, octyl dodecanol, cetostearyl alcohol, 2-decyltetradecinol, cholesterol, phytosterol, POE cholesterol ether, monostearyl glycerin ether (batyl alcohol), and monooleyl glyceryl ether (cerakyl alcohol).

Examples of the ester oils which can be used as (B) include diisobutyl adipate, 2-hexyldecyl adipate, di-2-heptylundecyl adipate, N-alkyl glycol monoisostearate, isocetyl isostearate, trimethylolpropane triisostearate, isononyl isononanate, isotridecyl isononanate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, octyldodecyl gum ester, oleyl oleate, octyldodecyl oleate, decyl oleate, neopentyl glycol dicaprirate, triethyl citrate, 2-ethylhexyl succinate, amyl acetate, ethyl acetate, butyl acetate, isocetyl stearate, butyl stearate, diisopropyl sebacinate, di-2-ethylhexyl sebacinate, cetyl lactate, myristyl lactate, isopropyl palmitate, 2-ethylhexyl palmitate, 2-hexyldecyl palmitate, 2-heptylundecyl palmitate, cholesteryl 12-hydroxystearate, dipentaerythritol fatty acid esters, isopropyl myristate, octyldodecyl myristate, 2-hexyldecyl myristate, myristyl myristate, hexyldecyl dimethyloctanoate, ethyl laurate, hexyl laurate, 2-octyldodecyl N-lauroyl-L-glutamate, and diisostearyl malate; and glyceride oils, e.g., acetoglyceryl, glycerol triisooctanoate, glyceryl triisostearate, glyceryl triisopalmitate, glyceryl monostearate, glyceryl di-2-heptylundecanoate, glyceryl trimyristate, and diglyceryl myristyl isostearate.

At least a part of the unctuous agent (B) is a linear or cyclic silicone oil represented by the formula, R⁶ₖSiO_{(4-k)/2}, wherein R⁶ is a hydrogen atom, a C₁₋₃₀ alkyl group, aryl group, aralkyl group, or fluorinated alkyl group and 0 ≤ k ≤ 2.5. Examples of the silicone oils which can be used as (B) include organopolysiloxanes having a low or high viscosity, such as dimethylpolysiloxane, methylphenylpolysiloxane, methylhydrogenpolysiloxane and dimethylsiloxane-methylphenylsiloxane copolymer; cyclosiloxanes, such as octamethylcyclotetrasiloxane, decamethylcyclopentasiloxane, dodecamethylcyclohexasiloxane, tetramethyltetrahydrogencyclotetrasiloxane and tetramethyl-tetraphenylcyclotetrasiloxane,tetramethyltetretrifluoropropyl cyclotetrasiloxane pentamethyltrifluoropropyl cyclopentasiloxane; silicone rubbers, such as gummy dimethylpolysiloxanes and gummy dimethylsiloxane-methylphenylsiloxane copolymers having high polymerization degrees; solutions of silicone rubbers in cyclosiloxane, trimethylsiloxysilicate, solutions of trimethylsiloxysilicate in cyclosiloxane, higher alkyl-modified silicones such as stearoxysilicone, alkyl-modified silicones, amino-modified silicones, fluorine-modified silicones, silicone resin and silicone resin solutions.

Examples of the fluorine-containing oils which can be used as (B) include perfluoropolyether, perfluorodecalin and perfluorooctane.

Depending on the form of cosmetics, it is preferred that 1 to 98 wt.% of unctuous agent (B), relative to the total cosmetics, may be added to cosmetics.

The cosmetics according to the present invention may contain water (C), depending on the aim of cosmetics. Suitable mixing ratio is 1 to 95 wt.% of the total cosmetics, depending on the form of the cosmetics.

The cosmetics according to the present invention may preferably contain one or more compounds having an alcoholic hydroxyl group in the molecular structure (D), depending on the aim of cosmetics. Examples of the compounds having an alcoholic hydroxyl group include lower alcohols such as ethanol and isopropanol; sugar alcohols such as sorbitol and maltose; sterols such as cholesterol, sitosterol, phytosterol, and lanosterol; and polyhydric alcohol such as glucose, butylene glycol, propylene glycol, dibuthylene glycol, and pentylene glycol. A desirable added amount ranges from 0.1 to 98 wt.% based on the total cosmetics.

Depending on the aim of cosmetics, the cosmetics according to the present invention may preferably contain one or more water-soluble or water-swelling polymer (E). Examples of such polymer include plant polymers such as gum Arabic, tragacanth gum, arabinogalactan, guar gum, karaya gum, carrageenan, pectin, agar, quince seed (i.e., marmelo), starch from rice, corn, potato or wheat, algae colloid, trant gum, and locust bean gum (carob gum); bacteria-derived polymers such as xanthan gum, dextran, succinoglucan, and pullulan; animal-derived polymers such as collagen, casein, albumin, and gelatin; starch-derived polymers such as carboxymethyl starch and methylhydroxypropyl starch; cellulose polymers such as methyl cellulose, ethyl cellulose, methylhydroxypropyl cellulose, carboxymethyl cellulose, hydroxymethyl cellulose, hydroxypropyl cellulose, nitrocellulose, sodium cellulose sulfate, sodium carboxymethyl cellulose, crystalline cellulose, and cellulose powder; alginic acid-derived polymers such as sodium alginate and propylene glycol alginate; vinyl polymers such as polyvinyl methylether, polyvinylpyrrolidone, and carboxyvinyl polymer; polyoxyethylene polymers such as polyoxyethylene/polyoxypropylene copolymers; acrylic polymers such as sodium polyacrylate, polyethyl acrylate, and polyacrylamide; other synthetic water-soluble polymers such as polyethyleneimine and cationic polymers; and inorganic water-soluble polymers such as, bentonite, aluminum magnesium silicate, montmorrilonite, beidellite, nontronite, saponite, hectorite, and silicic anhydride.

In these water-soluble polymers, film-forming agents, such as polyvinyl alcohol and polyvinyl pyrrolidine, are also included. Suitable amount to be contained is 0.1 to 25 wt.%, based on the total cosmetics.

The cosmetics according to the present invention may preferably contain one or more kinds of another powder and/or a coloring agent (F) in addition to the powder composition (A) and the dispersion of powder in oil.

Similarly to the powder that may be used for the powder composition (A) and the dispersion of powder in oil, the powder may be any powder that are commonly used in cosmetics, regardless of the shape, such as spherical, needle or plate, particle diameter such as smoke, fine particle, or pigment grade, and particle structure such as porous or non-porous. Examples of the powder include inorganic powder, organic powder, metal salt powder of surfactant, colored pigments, pearl pigments, metallic powder pigments, and natural colors.

These powders, of which examples have been already given above, may be the composite powder or powder which has been treated with general oil, silicone oil, fluorinated compounds, or surfactants as long as such treatment does not prevent the effect of the present invention and one or more kinds of these powders may be used. Suitable amount to be used is 0.1 to 99 wt.%, based on the total cosmetics. Especially for pressed powder cosmetics, suitable amount is 80 to 99 wt.% based on the total cosmetics.

The cosmetics according to the present invention may comprise one kind or two or more kinds of surfactant (G), depending on the aim of cosmetics. These surfactants has no particular restriction and may be any surfactants of anionic, cationic, nonionic or amphoteric surfactant, provided that it is commonly used in cosmetics.

Examples of the surfactants are as follows: the anionic surfactants include fatty acid soaps, such as sodium stearate and triethanolamine palmitate, alkylether carboxylic acids and salts thereof, salts of condensates of amino acids with fatty acids, alkyl sulfonate salts, alkenesulfonates, sulfonates of fatty acid esters, fatty acid amide sulfonates, sulfonate salts of the formalin condensates, salts of alkyl sulfates, salts of secondary higher alcohol sulfates, salts of alkyl/allyl ether sulfates, salts of fatty acid ester sulfates, salts of fatty acid alkylolamide sulfates, and salts of Turkey Red oil salfate, alkyl phosphate salts, ether phosphate salts, alkylallylether phosphate salts, amide phosphate salts, and N-acylamino surfactants; the cationic surfactants include amine salts such as alkylamine salts, amine salts of polyamine and amino alcohol fatty acid derivatives, alkyl quaternary ammonium salts, aromatic quaternary ammonium salts, pyridinium salts and imidazolium salts.

Examples of the nonionic surfactants include sorbitan fatty acid esters, glycerin fatty acid esters, polyglycerin fatty acid esters, propylene glycol fatty acid esters, polyethylene glycol fatty acid esters, sucrose fatty acid esters, polyoxyethylene alkyl ethers, polyoxypropylene alkyl ethers, polyoxyethylene alkyl phenyl ether, polyoxyethylene fatty acid esters, polyoxyethylene sorbitan fatty acid esters, polyoxyethylene sorbitol fatty acid esters, polyoxyethylene glycerin fatty acid esters, polyoxyethylene propylene glycol fatty acid esters, polyoxyethylene castor oil, polyoxyethylene hydrogenated castor oil, polyoxyethylene phytostanol ether, polyoxyethylene phytosterol ether, polyoxyethylene cholestanol ether, polyoxyethylene cholesteryl ether, linear or branched-polyoxyalkylene-modified organopolysiloxane, linear or branched polyoxyalkylene/alkyl-comodified organopolysiloxane, linear or branched-polyglycerin-modified organopolysiloxane, linear or branched-polyglycerin/alkyl-comodified organopolysiloxane, alkanolamide, sugar ethers, and sugar amides; and the amphoteric surfactants include betaine, aminocarboxylates, imidazoline derivatives, and amide amine type. A suitable amount of the surfactant to be added ranges from 0.1 to 20 wt.%, particularly preferably from 0.2 to 10 wt.% relative to the total amount of the cosmetic.

The cosmetics according to the present invention may contain one or more crosslinked organopolysiloxane (H), depending on the aim of the cosmetic. The cross-linking agent for the crosslinked organopolysiloxane preferably has two or more vinylic reactive sites, which react with hydrogen atoms directly bonded to silicon atoms to form crosslinked structure.

Additionally, this crosslinked organopolysiloxane preferably can absorb a larger amount of oil than that of itself to swell. Examples of the oils include silicone with a low viscosity from 0.65 mm²/sec to 10.0 mm²/sec, hydrocarbon oils and ester oils. It is also preferred to use a crosslinked organopolysiloxane containing, in the crosslinked molecule, at least a moiety selected from a group consisting of polyoxyalkylene, alkyl, alkenyl, aryl, and fluoroalkyl moieties.

Suitable amount of crosslinked organopolysiloxane to be added is preferably 0.1 to 50 wt.%, more preferably 1to 30 wt.%, based on the total cosmetics.

The cosmetics according to the present invention may contain one or more silicone resins, depending on the aim of the cosmetic.

The silicone resin is preferably acrylic silicone resin of acrylic/silicone graft or block copolymer. Use is also made of acrylic silicone resin containing in the molecule at least a moiety selected pyrrolidone moiety, long chain alkyl moiety, polyoxyalkylene moiety, fluoroalkyl moiety, and anionic moiety of carboxylic acid, etc.

Further, this silicone resin is preferably a silicone compound with network structure which is expressed as MQ, MDQ, MT, MDT, and MDTQ, wherein M is an R₃SiO_{1/2} unit,D is an R₂SiO unit,T is an RSiO_{3/2} unit and Q is an SiO₂ unit. Use is also made of silicone compounds with network structure which contain in the molecule at least a moiety selected from pyrrolidone moiety, long chain alkyl moiety, polyoxyalkylene moiety, fluoroalkyl moiety, and amino moiety.

For cosmetics comprising silicone resins such as acrylic silicone resin or silicone compound with network structure, suitable amount to be added is 0.1 to 20 wt.%, more preferably 1 to 10 wt.%, based on the total cosmetics.

In the cosmetic of the present invention, a variety of components that are commonly used in cosmetics can be blended in addition to the aforementioned components, as far as the purpose of the present invention is not damaged, for example, oil-soluble gelling agents, clay minerals modified with organic compounds, resins, antiperspirants, ultraviolet absorbents, ultraviolet absorbing and scattering agents, moisture retention agents, antiseptics, anti-microbial agents, fragrances, salts, antioxidants, pH regulators, a chelating agents, refreshing agents, an anti-inflammatory agent, skin beautifying components, such as skin whitener, cell activator, rough dry skin improver, blood circulation promoter, skin astringent and anti-seborrheic agent, vitamins, amino acids, nucleic acids, hormones, clathrate compounds, and hair setting agents.

The oil-soluble gelling agent may be a gelling agent selected from metal soaps, such as aluminum stearate, magnesium stearate and zinc myristate; amino acid derivatives, such as N-lauroyl-L-glutamic acid and a, γ-di-n-butylamine; dextrin fatty acid esters, such as dextrin palmitic acid ester, dextrin stearic acid ester and dextrin 2-ethylhexaminic acid palmitic acid ester; inulin fatty acid esters such as fructooligostearate; sucrose fatty acid esters, such as sucrose palmitic acid ester and sucrose stearic acid ester; benzylidene derivatives of sorbitol, such as monobenzylidene sorbitol and dibenzylidene sorbitol; and clay minerals modified with organic compounds, such as dimethylbenzyldodecyl ammonium montmorillonite clay and dimethyldioctadecyl ammonium montmorillonite clay.

Examples of the antiperspirant include aluminum chlorohydrate, aluminum chloride, aluminum sesquichlorohydrate, zirconium hydoxychloride, aluminum zirconium hydroxychloride, and aluminum zirconium glycine complex.

Examples of the ultraviolet absorbents include ultraviolet absorbents of benzoic acid type, such as p-aminobenzoic acid; those of anthranilic acid type, such as methyl anthranilate; those of salicylic acid type, such as methyl salicylate; those of succinic acid type, such as octyl p-methoxysuccinate; those of benzophenone type, such as 2,4-dihydroxybenzophenone; those of urocanic acid type, such as ethyl urocanate; and those of dibenzoylmethane type, such as 4-t-butyl-4'-methoxydibenzoylmethane. Examples of the ultraviolet absorbing and scattering agents include fine powder of titanium dioxide, fine powder of iron-containing titanium dioxide, fine powder of zinc oxide, fine powder of cerium oxide, and a mixture thereof.

Examples of the moisture retention agents include glycerin, sorbitol, propylene glycol, dipropylene glycol, 1,3-butylene glycol, pentylene glycol, glucose, xylitol, maltitol, polyethylene glycol, hyaluronic acid, chondroitin sulfuric acid, pyrrolidone carboxylate, polyoxyethylene glycoside, and polyoxypropylene methylglycoside.

For the antiseptics, alkyl paraoxybenzoates, benzoic acid, sodium benzoate, sorbic acid, potassium sorbate, and phenoxyethanol may be used. For the antibacterial agents, benzoic acid, salicylic acid, carbolic acid, sorbic acid, paraoxybenzoic acid alkyl esters, parachloromethacresol, hexachlorophene, benzalkonium chloride, chlorohexydine chloride, trichlorocarbanilide and phenoxyethanol.

Examples of the antioxidants include tocopherol, butylhydroxyanisole, dibutylhydroxytoluene and phytic acid; examples of the pH regulators include lactic acid, citric acid, glycolic acid, succinic acid, tartaric acid, dl-malic acid, potassium carbonate, sodium hydrogen carbonate and ammonium hydrogen carbonate; examples of the chelating agents include alanine, sodium ethylenediaminetetraacetate, sodium polyphosphate, sodium metaphosphate and phosphoric acid; examples of the refrigerants include L-menthol and camphor; and examples of the anti-inflammatory agents include allantoin, glycyrrhizin and salts thereof, glycyrrhetinic acid and stearyl glycyrrhetinate, tranexamic acid and azulene.

Examples of the skin-beautifying components include whitening agents, such as placenta extract, arbutin, glutathione and Yukinoshita extract; cell activators, such as royal jelly, photosensitizers, cholesterol derivatives and calf blood extract; rough and dry skin improvers; blood circulation improvers, such as nonylic acid vanillyl amide, benzyl nicotinate, beta -butoxyethyl nicotinate, capsaicin, zingerone, cantharis tincture, ichtammol, caffeine, tannic acid, alpha-borneol, tocopheryl nicotinate, inositol hexanicotinate, cyclandelate, cinnarizine, tolazoline, acetyl choline, verapamil, cepharanthin and gamma -oryzanol; skin astringents, such as zinc oxide and tannic acid; and anti-seborrheic agents, such as sulfur and thianthol.

Examples of the vitamins include vitamin A, such as vitamin A oil, retinol, retinyl acetate and retinyl palmitate; vitamin B, including vitamin B₂ such as riboflavin, riboflavin butyrate and flavin adenine nucleotide, vitamin B₆ such as pyridoxine hydrochloride, pyridoxine dioctanoate and pyridoxine tripalmitate, vitamin B₁₂ and its derivatives, and vitamin B15 and its derivatives; vitamin C, such as L-ascorbic acid, L-ascorbic acid dipalmitic ester, sodium (L-ascorbic acid)-2-sulfate and dipotassium L-ascorbic acid diphosphate; vitamin D, such as ergocalciferol and cholecarciferol; vitamin E, such as alpha - tocopherol, beta -tocopherol, gamma -tocopherol, dl- alpha -tocopheryl acetate, dl- alpha - tocopheryl nicotinate and dl- alpha -tocopheryl succinate; vitamin H; vitamin P; nicotinic acids, such as nicotinic acid, benzyl nicotinate and nicotinic acid amide; pantothenic acids, such as calcium pantothenate, D-pantothenyl alcohol, pantothenyl ethyl ether and acetylpantothenyl ethyl ether; and biotin.

Examples of the amino acids include glycine, valine, leucine, isoleucine, serine, threonine, phenylaranine, alginine, lysine, aspartic acid, glutamic acid, cystine, cysteine, methionine, and tryptophan; examples of the nucleic acids include deoxyribonucleic acid; and examples of the hormones include estradiol and ethenyl estradiol.

Examples of the polymers for hair setting include amphoteric, anionic, cationic, and nonionic polymers, such as polymers of polyvinyl pyrrolidone type such as polyvinyl pyrrolidone, vinyl pyrrolidone /vinyl acetate copolymers; acidic polymers of vinyl acetate ether type such as methyl vinyl ether / maleic acid anhydride alkyl half ester copolymer; polymers of acidic poly vinyl acetate type such as vinyl acetate / crotonic acid copolymer; acidic acrylic polymers such as (meth)acrylic acid / alkyl (meth) acrylate copolymer, (meth)acrylic acid / alkyl (meth) acrylate / alkyl acrylic amide copolymer, and amphoteric acrylic polymer such as N-methacryloylethyl-N,N-dimethylammonium alpha-N-methylcarboxybetaine / alkylmetahcrylate copolymer, hydroxypropyl (metha)acrylate/butylaminoethyl methacrylate / octyl amide of acrylic acid copolymer. Use is also made of naturally occurring polymers such as cellulose or derivatives thereof, keratin, collagen and derivatives thereof.

The term "cosmetic materials" as used herein are intended to include skin care products, such as face lotion, milky lotion, cream, face cleansing cream, massage materials, toilet soap and detergent, antiperspirant and deodorant; makeup products, such as face powder, foundation, rouge, eye shadow, mascara, eyeliner and lipstick; and hairdressing products, such as shampoo, rinse, treatment setting agent, antipersipirant and ultraviolet protection cosmetics, such as sunscreen milky lotion or sunscreen cream.

Additionally, the present cosmetic materials may have various forms such as liquid, emulsion, solid, paste, gel, powder, press, laminate, mousse, spray, stick, pencil forms.

The present invention will be further explained in detail below by referring to the Examples and the Comparative Examples. However, the present invention shall not be limited to these examples. "%" described below implies "% by weight" unless otherwise specified.

### EXAMPLES

### Preparation Example 1

To a reaction vessel were placed 478 parts by weight of organohydrogen siloxane according to the following formula (6), 200 parts by weight of isopropyl alcohol, 21 parts by weight of diglycerin monoallyl ether according to the following formula (7), and 302 parts by weight of silicone modified with a vinyl group at one end according to the formula (8). Then 2 parts of a 0.5 wt.% chloroplatinic acid solution solution in isopropyl alcohol were added to react for 6 hours under reflux of solvent.

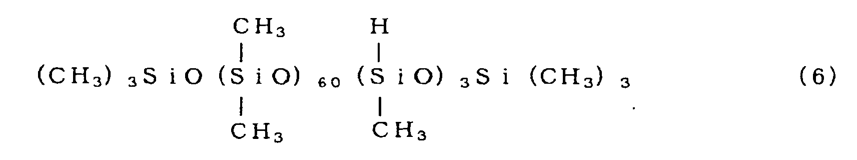

CH₂=CHCH₂OCH₂CH(OH)CH₂OCH₂CH(OH)CH₂OH (7)

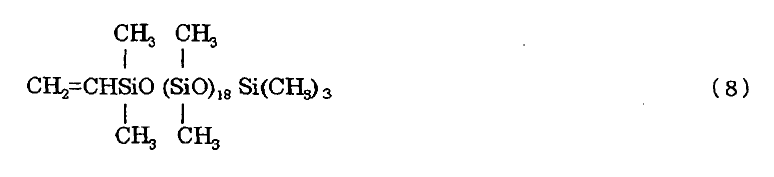

The reaction mixture was heated under reduced pressure to distill the solvent off to obtain organopolysiloxane according to the following formula (9). The product was a transparent pale-brown liquid with the viscosity of 9500 mm²/sec at 25 degree C.

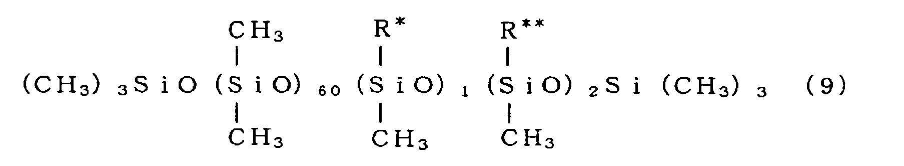

R*= C₃H₆OCH₂CH(OH)CH₂OCH₂CH(OH)CH₂OH

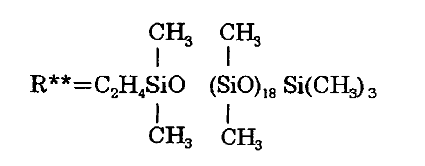

### Preparation Example 2

To a reaction vessel were placed 234 parts by weight of organohydrogen siloxane according to the following formula (10), 150 parts by weight of isopropyl alcohol, 26 parts by weight of triglycerin monoallyl ether according to the following formula (11), and 168 parts by weight of silicone modified with a vinyl group at one end according to the formula (12). Then 2 parts of 0.5 wt.% chloroplatinic acid solution in isopropyl alcohol were added to react for 6 hours under reflux of solvent. 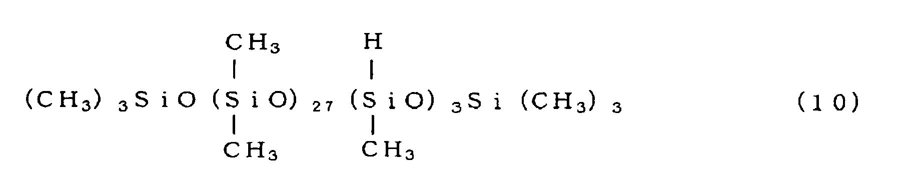 CH₂=CHCH₂OCH₂CH(OH)CH₂OCH₂CH(OH)CH₂OCH₂CH(OH)CH₂OH (11)

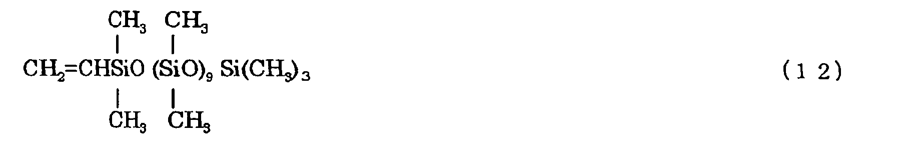

The reaction mixture was heated under reduced pressure to distill the solvent off to obtain organopolysiloxane according to the following formula (13). The product was a transparent pale-brown liquid with the viscosity of 5300 mm²/sec at 25 degree C.

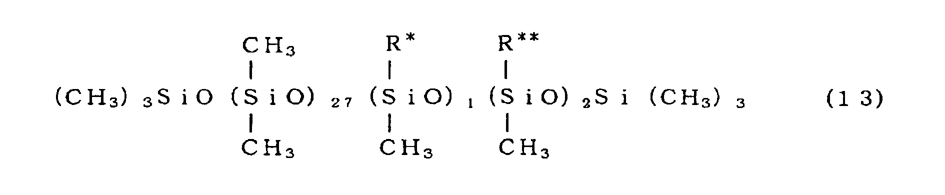

R*= C₃H₆OCH₂CH(OH)CH₂OCH₂CH(OH)CH₂OCH₂CH(OH)CH₂OH

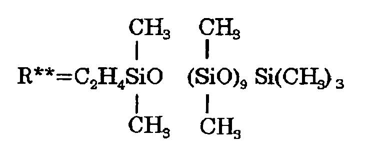

### Preparation Example 3

To a reaction vessel were placed 120 parts by weight of organohydrogen siloxane according to the following formula (15), 200 parts by weight of isopropyl alcohol, 28 parts by weight of triglycerin monoallyl ether according to the above formula (11), 84 parts by weight of silicone modified with a vinyl group at one end according to the above formula (12), and 51 parts by weight of 1-dodecene. Then 2 parts of 0.5 wt.% chloroplatinic acid solution in isopropyl alcohol were added to react for 6 hours under reflux of solvent.

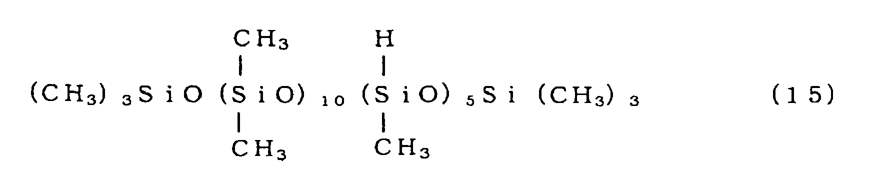

The reaction mixture was heated under reduced pressure to distill the solvent off to obtain organopolysiloxane according to the following formula (16). The product was a transparent pale-brown liquid with the viscosity of 7000 mm²/sec at 25 degree C.

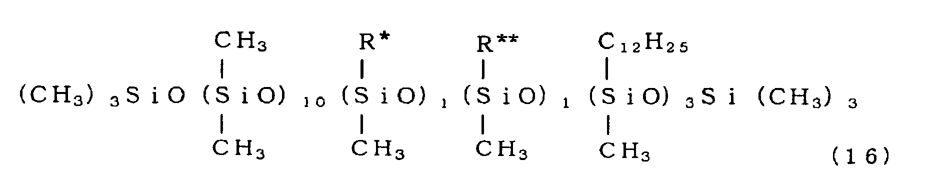

R*= C₃H₆OCH₂CH(OH)CH₂OCH₂CH(OH)CH₂OCH₂CH(OH)CH₂OH

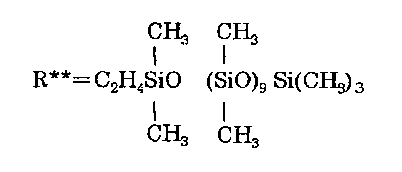

### Preparation Example 4

To a reaction vessel were placed 272 parts by weight of organohydrogen siloxane according to the following formula (17), 200 parts by weight of isopropyl alcohol, 53 parts by weight of triglycerin monoallyl ether according to the above formula (11), and 252 parts by weight of organopolysiloxane according to the formula (12). Then 2 parts of 0.5 wt.% chloroplatinic acid solution in isopropyl alcohol was added to react for 6 hours under reflux of solvent.

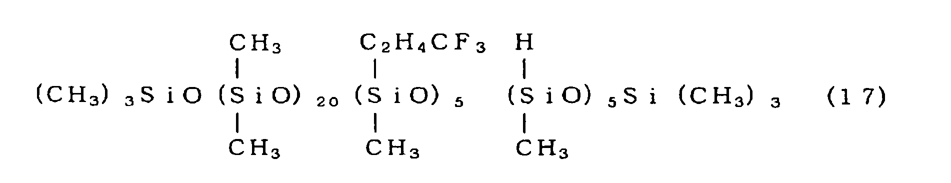

The reaction mixture was heated under reduced pressure to distill the solvent off to obtain organopolysiloxane according to the following formula (18). The product was a transparent pale-brown liquid with the viscosity of 14000 mm²/sec at 25 degree C.

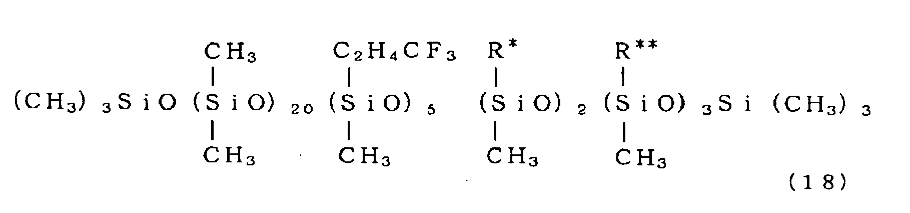

R*= C₃H₆OCH₂CH(OH)CH₂OCH₂CH(OH)CH₂OCH₂CH(OH)CH₂OH

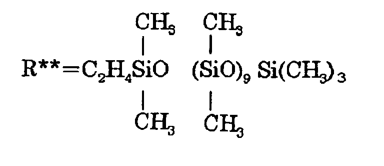

### Preparation Example 5

To a reaction vessel were placed 478 parts by weight of organohydrogen siloxane according to the above formula (19), 300 parts by weight of isopropyl alcohol, 36 parts by weight of the compound according to the following formula (20), and 168 parts by weight of the compound according to the above formula (12). Then 2 parts of 0.5 wt.% chloroplatinic acid solution in isopropyl alcohol were added to react for 6 hours under reflux of solvent.

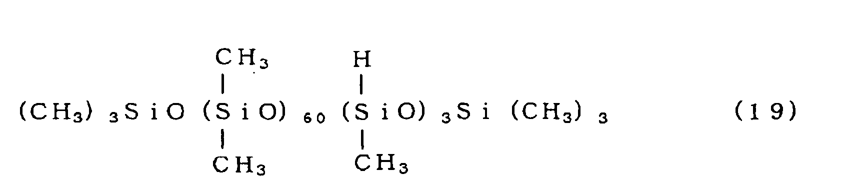

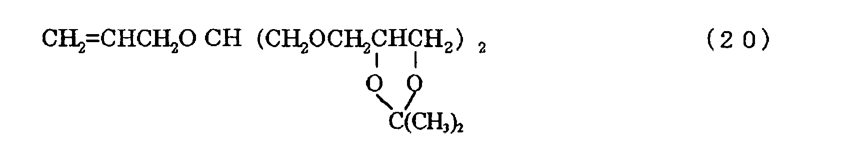

Further 100 g of 0.025 N hydrochloric acid was added and then deacetonization was carried out for 6 hours under reflux. The reaction mixture was heated under reduced pressure to distill the solvent off to obtain organopolysiloxane according to the following formula (21). The product was a transparent pale-brown liquid with the viscosity of 12000 mm²/sec at 25 degree C.

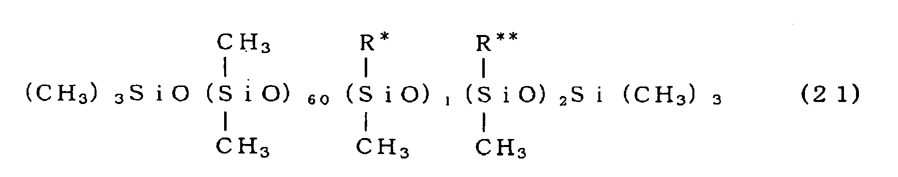

R*=C₃H₆OCH(CH₂OCH₂CH(OH)CH₂OH)₃

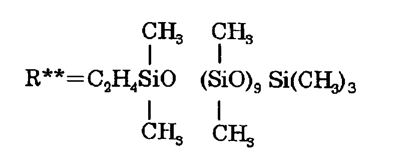

### Example 1

Ten grams of organopolysiloxane from the above-described Preparation Example 1 was dissolved in 50 g of decamethylcyclopentasiloxane. Then 40 g of titanium dioxide, MT-100TV, from Teika, Co., Ltd., was added and dispersed with the aid of a beads mill to obtain a dispersion of titanium dioxide (a).

### Example 2

Eight grams of organopolysiloxane from the above-described Preparation Example 2 was dissolved in 42 g of decamethylcyclopentasiloxane. Then 50 g of zinc oxide, MZ505S, from Teika, Co., Ltd., was added and dispersed with the aid of a beads mill to obtain a dispersion of zinc oxide (b).

### Example 3

Ten grams of organopolysiloxane from the above-described Preparation Example 3 was dissolved in 40 g of decamethylcyclopentasiloxane. Then 50 g of zinc oxide, MZ505S, from Teika, Co., Ltd., was added and dispersed with the aid of a beads mill to obtain a dispersion of zinc oxide (c).

### Example 4

Eight grams of organopolysiloxane from the above-described Preparation Example 4 was dissolved in 52 g of decamethylcyclopentasiloxane. Then 40 g of titanium dioxide, MT-100TV, from Teika, Co., Ltd., was added and dispersed with the aid of a beads mill to obtain a dispersion of titanium dioxide (d).

### Comparative Example 1

Ten grams of polyether-modified silicone, KF6017, from Shin-Etsu Chemical Co., Ltd., was dissolved in 40 g of decamethylcyclopentasiloxane. Then 50 g of zinc oxide, MZ505S, from Teika, Co., Ltd., was added and dispersed with the aid of a beads mill to obtain a dispersion of zinc oxide (e).

### Comparative Example 2

Ten grams of organopolysiloxane from the above-described Preparation Example 5 was dissolved in 40 g of decamethylcyclopentasiloxane. Then 40 g of titanium dioxide, MT-100TV, from Teika, Co., Ltd., was added and dispersed with the aid of a beads mill to obtain a dispersion of titanium dioxide (f).

### Example 5

Five grams of organopolysiloxane from the above-described Preparation Example 1 was dissolved in isopropyl alcohol. Then 40 g of titanium dioxide, MT-100TV, from Teika, Co., Ltd., was added and dispersed. The solvent was distilled off to obtain a titanium dioxide composition (g).

### Example 6

Five grams of organopolysiloxane from the above-described Preparation Example 2 was dissolved in isopropyl alcohol. Then 50g of zinc oxide, MZ505S, from Teika, Co., Ltd., was added and dispersed. Then the solvent was distilled off to obtain a zinc oxide composition (h).

### Example 7

Eight grams of organopolysiloxane from the above-described Preparation Example 3 was dissolved in isopropyl alcohol. Then 50 g of zinc oxide, MZ505S, from Teika, Co., Ltd., was added and dispersed. The solvent was distilled off to obtain a zinc oxide composition (i).

### Example 8

Ten grams of organopolysiloxane from the above-described Preparation Example 4 was dissolved in isopropyl alcohol. Then 40 g of titanium dioxide, MT-100TV, from Teika, Co., Ltd., was added and dispersed. Then the solvent was distilled off to obtain a zinc oxide composition (j).

### Comparative Example 3

Ten grams of polyether-modified silicone, KF6017, from Shin-Etsu Chemical Co., Ltd., was dissolved in isopropyl alcohol. Then 50 g of zinc oxide, MZ505S, from Teika, Co., Ltd., was added and dispersed. Then the solvent was distilled off to obtain a zinc oxide composition (k).

### Comparative Example 4

Ten grams of organopolysiloxane from the above Preparation Example 5 was dissolved in 5 g of isopropyl alcohol. Then 40 g of titanium dioxide, MT-100TV, from Teika, Co., Ltd., was added. The solvent was distilled off to obtain a titanium dioxide composition (1).

### Evaluation of Dispersibility

Each sample from the dispersions of powder and the powder compositions from Examples 1 to 8 and Comparative Examples 1 to 4 was mixed with decamethylcyclopentacyclohexane in a concentration of the powder of 5 % and the mixture was put in a 50ml tube settler and was allowed to settle. The ratio of the sediment height to the whole liquid phase height was determined visually two day later. The results are as shown in the table below.

| | Sediment height /whole liquid phase height, % | | Sediment height /whole liquid phase height, % |
|---|---|---|---|
| Ex. 1 | 0.3 | Ex. 7 | 0.8 |
| Ex. 2 | 0.6 | Ex. 8 | 0.9 |
| Ex. 3 | 0.7 | Com. Ex. 1 | 7.5 |
| Ex. 4 | 0.3 | Com. Ex. 2 | 0.8 |
| Ex. 5 | 0.5 | Com. Ex. 3 | 8.5 |
| Ex. 6 | 0.8 | Com. Ex. 4 | 0.5 |

In Examples 1 to 8 and Comparative Examples 2 and 4, the dispersions are remained homogeneous with little settling observed, and therefore their dispersibility was good. However, in Comparative Examples 1 and 3, the dispersions were unhomogeneous and substantial settling was observed.

### Examples 9 to 16 and Comparative Examples 5 and 6

Sunscreen agent was prepared in the formulation shown in Table 1 to evaluate the product quality. The unit is "part".

**Table 1**

| | Example | | | | | | | | Comparative Ex. | |
|---|---|---|---|---|---|---|---|---|---|---|
| | 9 | 10 | 11 | 12 | 13 | 14 | 15 | 16 | 5 | 6 |
| 1 KF96 6cs | 5.0 | 5.0 | 5.0 | 5.0 | 5.0 | 5.0 | 5.0 | 5.0 | 5.0 | 5.0 |
| 2 KSG-210 | 5.0 | 5.0 | 5.0 | 5.0 | 5.0 | 5.0 | 5.0 | 5.0 | 5.0 | 5.0 |
| 3 Glyceryl triisooctate | 3.0 | 3.0 | 3.0 | 3.0 | 3.0 | 3.0 | 3.0 | 3.0 | 3.0 | 3.0 |
| 4 KF-6019 | 1.0 | 1.0 | 1.0 | 1.0 | 1.0 | 1.0 | 1.0 | 1.0 | 1.0 | 1.0 |
| 5 Octyl p-methoxycinnamate | 6.0 | 6.0 | 6.0 | 6.0 | 6.0 | 6.0 | 6.0 | 6.0 | 6.0 | 6.0 |
| 6 Sodium chloride | 0.5 | 0.5 | 0.5 | 0.5 | 0.5 | 0.5 | 0.5 | 0.5 | 0.5 | 0.5 |
| 7 1,3-butylene glycol | 2.0 | 2.0 | 2.0 | 2.0 | 2.0 | 2.0 | 2.0 | 2.0 | 2.0 | 2.0 |
| 8 Decamethylcyclopentasiloxane | | | | | 30 | 25 | 30 | 30 | | 25 |
| 9 Purified water | balance | balance | balance | balance | balance | balance | balance | balance | balance | balance |
| 10 Perfume | proper dose | proper dose | proper dose | proper dose | proper dose | proper dose | proper dose | proper dose | proper dose | proper dose |
| 11 Dispersion of titaniun oxide (a) [Example 1] | 50 | | | | | | | | | |
| 12 Dispersion of zinc oxide (b) [Example 2] | | 50 | | | | | | | | |
| 13 Dispersion of zinc oxide (c) [Example 3] | | | 50 | | | | | | | |
| 14 Dispersion of titanium oxide (d) [Example 4] | | | | 50 | | | | | | |
| 15 Composition of titanium oxide (g) [Example 5] | | | | | 20 | | | | | |
| 16 Composition of zinc oxide (h) [Example 6] | | | | | | 25 | | | | |
| 17 Composition of zinc oxide (i) [Example 7] | | | | | | | 20 | | | |
| 18 Composition of titanium oxide (j) [Example 8] | | | | | | | | 20 | | |
| 19 Dispersion of zinc oxide (e) [Comp. Ex. 1] | | | | | | | | | 50 | |
| 20 Composition of titanium oxide (I) [Comp. Ex. 4] | | | | | | | | | | 25 |
| Evaluation Results | | | | | | | | | | |
| 1. Stability of dispersion | ++ | ++ | ++ | ++ | ++ | ++ | ++ | ++ | | ++ |
| 2. Users' satisfaction | | | | | | | | | | |
| Light and smooth feeling | ++ | ++ | ++ | ++ | ++ | + | ++ | ++ | ∓ | ++ |
| Spreadability | ++ | + | ++ | ++ | ++ | ++ | ++ | ++ | | + |
| Transparency of cosmetic films | ++ | ++ | ++ | + | ++ | ++ | ++ | ++ | | ++ |
| Non-stickiness | ++ | ++ | ++ | ++ | ++ | ++ | ++ | + | | ++ |
| Protecting effect against sun burn | ++ | ++ | ++ | ++ | ++ | ++ | ++ | ++ | ∓ | ++ |
| Scent | ++ | + | + | ++ | ++ | ++ | ++ | ++ | | |

| | | | | | | | | | | |
|---|---|---|---|---|---|---|---|---|---|---|
| KF-96: dimethylpolysiloxane. KSG-210: crosslinked polyether-modified silicone, KF-6109 : polyethersilicone from Shin-Etau Chemical Co., Ltd. | | | | | | | | | | |

### Preparation Procedures of Sunscreen Agent

### In the case of Examples 9 to 12 and Comparative Example 5

A: Components 1,2,3,and 4 were mixed homogeneously.
B: Components 6,7, and 9 were mixed homogeneously.
C: B was added to A to emulsify.
D: Components 5, 10, 11 to 14, and 19 were added to C to obtain a sunscreen agent.

### In the case of Example 13 to 16 and Comparative Example 6

A: Components 1,2,3,and 4 were mixed homogenously and components 8,10,15 to 18, and 20 were added.
B: Components 6,7, and 9 were mixed homogenously.
C: B was added to A and the combined mixture was emulsified.
D: Component 5 was added to C to obtain a sunscreen agent.

The evaluations of products quality as described in Table 1 were according to the following procedures.

### 1. Stability of Dispersion of Powder

After allowing sunscreen agents to stand sill at room temperature for a month, the aggregation of powder was observed to determine the stability of the dispersion according to the following criteria.
Evaluation criteria + +: aggregable property is not observed.
+: aggregation of powder is observed slightly.
-: tendency to aggregate is observed.
--: aggregation of powder is clearly observed.

### 2. User's Satisfaction

The obtained sunscreen agents were evaluated concerning dry touch feeling, spreadability, transparency of cosmetic films, stickiness to skin, sun burn protecting effect, and scent by 50 women panelists using 5 scales of the following criteria. Based on the obtained average score, each sunscreen agent was given grade, such as + or -, according to the following criteria.

| | |
|---|---|
| Criteria for Evaluation | |
| 5 points | good |
| 4 points | slightly good |
| 3 points | ordinary |
| 2 points | slightly bad |
| 1 point | bad |

| | |
|---|---|
| Evaluation of average point | |
| If the average score is 4.5 or higher | + + |
| If the average score is not lower than 3.5 but not higher than 4.5 | + |
| If the average score is not lower than 2.5 but not higher than 3.5 | - |
| If the average score is not lower than 1.5 but not higher than 2.5 | - - |

As is clear from the results of Table 1, the sunscreen agents of Examples 9 to 16 did not show aggregation and had a good dispersibility. The users satisfaction was also good in every item. Contrary to this, in the sunscreen agent of the Comparative Example 5 where polyethersioicone was added, a slight aggregation was observed, the transparency of the cosmetic film was inferior, and a sufficient user satisfaction was not obtained. In the sunscreen agent of Comparative Example 6, aggregation was not observed and a good dispersibility and a good user satisfaction was obtained but it had a specific scent.

### Example 17: Oil-in-water Type Cream

| Component | Weight % |
|---|---|
| 1. Ethanol | 17.0 |
| 2. Propylene glycol | 3.0 |
| 3. Polyether-modified silicone ¹⁾ | 0.5 |
| 4. Glyceryl trioctanoate | 2.0 |
| 5. Sericite treated with organopolysiloxane from Preparation Example 1 ²⁾ | 3.0 |
| 6. Composite powder of hybrid silicone ³⁾ | 5.0 |
| 7. Carboxyvinyl polymer (1% aqueous solution) | 20.0 |
| 8. Xanthan gum (2% aqueous solution) | 6.0 |
| 9. Triethanolamine | 0.2 |
| 10. Antiseptics agent | q.l. |
| 11. Fragrance | q.l. |
| 12. Purified water | 60.8 |

| | |
|---|---|
| 1) Polyether-modified silicone; KF-6011, from Shin-Etsu Chemical Co., Ltd. 2)Sericite treated with organopolysiloxane; obtained by dissolving 2 g organopolysiloxane of the above Preparation Example 1 in isopropyl alcohol, dispersing 98 g of sericite, distilling the solvent off, and ripening under heat. 3) Composite powder of hybrid silicone; K S P - 1 0 0, from Shin-Etsu Chemical Co., Ltd. | |

### Preparation Procedures

A: Components 1 to 6 were mixed.
B: Components 7 to 12 were mixed to dissolve.
C: A was added to B and the resulting mixture was emulsified by stirring.

The oil-in-water type cream thus obtained was found to have a fine texture with non-sticky, non-oily, moisturizing, and hydrating touch and spread lightly. It could also provide cool feeling for skin and makeup coverage lasted long. In addition, it was found that the present oil-in-water type cream did not cause quality change with temperature and time, having a very excellent stability.

### Example 18 : Oil-in-water Cream

| Component | Weight % |
|---|---|
| 1. Crosslinked dimethylpolysiloxane ¹⁾ | 10.0 |
| 2 Glyceryl trioctanoate | 5.0 |
| 3. Dipropylene glycol | 7.0 |
| 4. Glycerin | 5.0 |
| 5. Methyl cellulose (2% aqueous solution) ²⁾ | 7.0 |
| 6. Emulsifier of polyacrylic amide type ³⁾ | 2.0 |
| 7.Mica titanium treated with organopolysiloxane from Preparation Example 2 ⁴⁾ | 1.0 |
| 8.Antiseptics | q.l. |
| 9.Fragrance | q.l. |
| 10.Purified water | 63.0 |

| | |
|---|---|
| 1) Crosslinked dimethylpolysiloxane; KSG-16 from Shin-Etsu Chemical Co., Ltd. 2) Methyl cellulose; Metholose SM-4000 from Shin-Etsu Chemical Co., Ltd. 3) Emulsifier of polyacrylic amide type; Sepigel 305 from SEPIC 4) Mica titanium obtained by dissolving 2 g of organopolysiloxane of Preparation Example 2 in isopropyl alcohol, adding 98 g of mica titanium to disperse, and distilling the solvent off. | |

### Preparation Procedures

A: Components 3 to 10 were mixed.
B: Components 1 and 2 were mixed to dissolve and A was added. The resulting mixture was emulsified by stirring.

The oil-in-water type cream thus obtained was found to have a fine texture with non-sticky, non-oily, moisturizing, and hydrating touch and spread lightly. It could also provide cool feeling for skin and makeup coverage lasted long. In addition, it was found that the present oil-in-water type cream did not cause quality change with temperature and time, having a very excellent stability.

### Example 19: Water-in-oil Type Cream

| Component | | Weight % |
|---|---|---|
| 1. | Dimethylpolysiloxane (6 mm²/ sec at 25 degree C) | 6.0 |
| 2. | Methylphenylpolysiloxane | 4.0 |
| 3. | Squalane | 5.0 |
| 4. | Neopentylglycol dioctanoate | 3.0 |
| 5. | Polyether-modified silicone ¹⁾ | 3.0 |
| 6. | Fine particulate of hydrophobized titanium dioxide ²⁾ | 2.0 |
| 7. | Magnesium sulfate | 0.7 |
| 8. | Glycerin | 10.0 |
| 9. | Antiseptics | q.l. |
| 10. | Fragrance | q.l. |
| 11. | Purified water | balance |

| | | |
|---|---|---|
| 1) Polyether-modified silicone; KF 6012 from Shin-Etsu Co., Ltd. 2) Fine particulate of hydrophobized titanium powder; fine particulate of titanium dioxide with average particulate diameter of 0.05 pm was dispersed in water so that the content of titanium would be 10 wt.%. Then 10 wt.% sodium silicate solution, where the molar ratio of SiO₂/Na₂O = 0.5, was added so that the SiO₂ content would be 2 wt.% relative to titanium dioxide and 10 wt. % aluminum sulfate solution was added dropwise so that the Al₂O₃ content would be 7.5 wt.% relative to titanium dioxide to deposit silicic acid hydrate and alumina hydrate on the surface of titanium dioxide. After the reaction was completed, the reactant was filtered, washed, dried and pulverized with the aid of jet mill. The resulting particulate was placed in Henschel mixer, 2 wt.% of organopolysiloxane from Preparation Example 3 was added while stirring sufficiently, the resulting mixture was mixed and stirred, and then was calcined at 120 degree C. | | |

### Preparation Procedures

A: Components 1 to 5 were mixed while heating and then component 6 was added. The resulting mixture was mixed homogenously.
B: Components 7 to 9 and 11 were dissolved while heating.
C: While stirring, B was added dropwise to A. The resulting mixture was emulsified, cooled, and component 10 was added thereto to obtain cream.

The water-in-oil type cream thus obtained was found to have a fine texture with non-sticky, non-oily, moisturizing, and hydrating touch and spread lightly. It could also provide cool feeling for skin and makeup coverage lasted long. In addition, it was found that the present water-in-oil type cream did not cause quality change with temperature and time, having a very excellent stability.

### Example 20: Water-in-oil Type Cream

| Component | | Weight % |
|---|---|---|
| 1. | Alkyl-modified crosslinked polyether-modified silicone ¹⁾ | 6.0 |
| 2. | Liquid paraffins | 13.5 |
| 3. | Macadamia nuts oil | 5.0 |
| 4. | Alkyl/polyether co-modified silicone ²⁾ | 0.5 |
| 5. | Composite powder of hybrid silicone ³⁾ | 3.0 |
| 6. | Dispersion of titanium dioxide ⁴⁾ | 2.0 |
| 7. | Sodium citrate | 0.2 |
| 8. | Propylene glycol | 8.0 |
| 9. | Glycerin | 3.0 |
| 10. | Antiseptics | q.l. |
| 11. | Fragrance | q.l. |
| 12. | Purified water | 58.8 |

| | | |
|---|---|---|
| 1) Alkyl-modified crosslinked polyether modified silicone KSG-310 from Shin-Etsu Co., Ltd. 2) Alkyl/polyether co-modified silicone; KF-6026 from Shin-Etsu Co., Ltd. 3) Composite powder of hybrid silicone; KSP-100 from Shin-Etsu Co., Ltd. 4) Dispersion of titanium dioxide: obtained by dissolving 10 g or organopolysiloxane of the above Preparation Example 3 in 50 g of tridecyl isononanate, adding 40 g of titanium dioxide, MV-100TV from Teika Co., Ltd., thereto, and dispersing the resulting mixture with the aid of beads mill to obtain the dispersion of titanium dioxide. | | |

### Preparation Procedures

A: Components 1 to 6 were mixed.
B: Components 7 to 12 were mixed to dissolve and the resulting mixture was added to A. The resulting mixture was emulsified.

The water-in-oil type cream thus obtained was found to have a fine texture with non-sticky, non-oily, moisturizing, and hydrating touch and spread lightly. It could also provide cool feeling for skin and makeup coverage lasted long. In addition, it was found that the present water-in-oil type cream did not cause quality change with temperature and time, having a very excellent stability.

### Example 21: Water-in-oil Type Cream

| Component | | Weight % |
|---|---|---|
| 1. | Dimethylpolysiloxane (6 mm²/sec at 25 degree C) | 4.0 |
| 2. | Polyether-modified silicone ¹⁾ | 5.0 |
| 3. | POE (5) octyl dodecyl ether | 1.0 |
| 4. | Polyoxyethylene sorbitan monostearate (20E.O.) | 0.5 |
| 5. | Zinc oxide treated with silicic anhydride ²⁾ | 2.0 |
| 6. | Dispersion of titanium dioxide of Example 1 (A) | 25.0 |
| 7. | Liquid paraffins | 2.0 |
| 8. | Macadamia nuts oil | 1.0 |
| 9. | Scuttellaria Root Extract³⁾ | 1.0 |
| 10. | Gentiana Extract⁴⁾ | 0.5 |
| 11. | Ethanol | 5.0 |
| 12. | 1,3-Buthylene glycol | 2.0 |
| 13. | Antiseptics | q.l. |
| 14. | Fragrance | q.l. |
| 15. | Purified water | balance |

| | | |
|---|---|---|
| 1) Polyether-modified silicone; KF6019 from Shin-Etsu Co., Ltd. 2) Zinc oxide treated with silicic anhydride: slica with a particle size ranging from 0.01 to 10µm, containing 50% of zinc oxide; SUNSPHERE SZ-5 from Asahi Glass Company. 3) Scuttellaria Root Extract; extracted with a 50% aqueous 1,3-butylene glycol solution. 4) Gentiana Extract: extracted with a 20% aqueous ethanol solution. | | |

### Preparation Procedures

A: Components 5 to 8 were mixed and the resulting mixture was dispersed homogeneously.
B: Components 1 to 4 were mixed and A was added.
C: Components 9 to 14 and 16 were mixed and B was added. The resulting mixture was emulsified.
D: C was cooled and component 15 was added to obtain cream.

The water-in-oil type cream thus obtained was found to have a fine texture with non-sticky, non-oily, moisturizing, and hydrating touch and spread lightly. It could also provide cool feeling for skin and makeup coverage lasted long. In addition, it was found that the present water-in-oil type cream did not cause quality change with temperature and time, having a very excellent stability.

### Example 22: Eyeliner

| Component | | Weight % |
|---|---|---|
| 1. | Decamethylpentasiloxane | 14.0 |
| 2. | Polyether-modified silicone ¹⁾ | 3.0 |
| 3. | Organosilicone resin ²⁾ | 15.0 |
| 4. | Montmorillonite modified with dioctadecyldimethylammonium salt | 3.0 |
| 5. | Dispersion of iron oxide black ³⁾ | 25.0 |
| 6. | 1.3-Butylene glycol | 5.0 |
| 7. | Sodium dehydroacetate | q.l. |
| 8. | Antiseptics | q.l. |
| 9. | Purified water | balance |

| | | |
|---|---|---|
| 1) Polyether-modified silicone; KF6017 from Shin-Etsu Co., Ltd. 2) Organosilicone resin; KF-7312J from Shin-Etsu Co., Ltd. 3) Dispersion of iron oxide black; Iron oxide black 40% Organopolysiloxane of Preparation Example 4 10 % Dispersion obtained by dispersing decamethylpentasilixoane with the aid of beads mill 50% | | |

### Preparation Procedures

A: Components 1 to 4 were mixed and component 5 was added. The resulting mixture was dispersed homogeneously.
B: Components 6 to 9 were mixed.
C: B was added dropwise to A and the mixture was emulsified to obtain eyeliner.

The eyeliner thus obtained demonstrated a light spreadability and was easy to draw with. It also gave a refreshing and non oily feelings to users. No quality change was found with temperature and time and it showed superior stability and good users' satisfaction. It was also found that cosmetic effect maintained long with excellent water resistance and sweat resistance.

### Example 23: Foundation

| Component | | Weight % |
|---|---|---|
| 1. | Decamethylcyclopentasiloxane | 45.0 |
| 2. | Dimethylpolysiloxane (6 mm²/sec at 25 degree C) | 5.0 |
| 3. | Crosslinked polyether-modified silicone ¹⁾ | 3.0 |
| 4. | Polyether-modified silicone ²⁾ | 1.0 |
| 5. | Montmorillonite modified with octadecyldimethylbenzylammonium salt | 4.0 |
| 6. | Titanium dioxide treated with organopolysiloxane from Preparation Example 1³⁾ | 10.0 |
| 7. | Talc treated with organopolysiloxane from Preparation Example 1³⁾ | 6.0 |
| 8. | Mica treated with organopolysiloxane from Preparation Example 1³⁾ | 6.0 |
| 9. | Iron oxide red treated with organopolysiloxane from Preparation Example 1³⁾ | 1.6 |
| 10. | Iron oxide yellow treated with organopolysiloxane fromPreparation Example 1³⁾ | 0.7 |
| 11. | Iron oxide black treated with organopolysiloxane from Preparation Example 1 ³⁾ | 0.2 |
| 12. | Dipropylene glycol | 5.0 |
| 13. | Methyl paraoxybenzoate | 0.3 |
| 14. | 2-amino-2-methyl-1,3-propanediol | 0.2 |
| 15. | Hydrochloric acid | 0.1 |
| 16. | Fragrance | q.l. |
| 17. | Purified water | q.l. |

| | | |
|---|---|---|
| 1) Crosslinked olyether-modified silicone; KSG-210 from Shin-Etsu Chemical Co., Ltd. 2) Polyether-modified silicone; KF-6019 from Shin-Etsu Chemical Co., Ltd. 3) Treated powder; obtained by dissolving 2 g of organopolysiloxane of Preparation Example 1 in isopropyl alcohol, adding 98 g of each powder, dispersing the resulting mixture and distilling the solvent off therefrom. | | |

### Preparation Procedures

A: Components 1 to 5 were mixed while heating and components 6 to 11 were added. The resulting mixture was made homogeneous.
B: Components 12 to 15 and 17 were dissolved with heating while the pH of the aqueous phase was kept at 9.0.
C: While stirring, B was added dropwise to A to emulsify. The resulting emulsion was cooled and the component 16 was added to obtain foundation.

The foundation thus obtained was found to have a fine texture with non-sticky, non-oily, moisturizing, and hydrating touch and spread lightly. It could also provide cool feeling for skin and makeup coverage lasted long. In addition, it was found that the present foundation did not cause quality change with temperature and time, having a very excellent stability.

### Example 24: Cream Eyeshadow

| Component | | Weight % |
|---|---|---|
| 1. | Decamethylcyclopentasiloxane | 15.0 |
| 2. | Dimethylpolysiloxane (6 mm²/sec at 25 degree C) | 10.0 |
| 3. | Branched polyether-modified silicone ¹⁾ | 2.0 |
| 4. | PEG (10) lauryl ether | 0.5 |
| 5. | Cromium oxide treated with organopolysiloxane from Preparation Example 2 ²⁾ | 6.2 |
| 6. | Ultramarine blue treated with organopolysiloxane from Preparation Example 2 ²⁾ | 4.0 |
| 7. | Mica coated with titanium treated with organopolysiloxane from Preparation Example 2 ²⁾ | 6.0 |
| 8. | Sodium chloride | 2.0 |
| 9. | Propylene glycol | 8.0 |
| 10. | Antiseptics | q.l. |
| 11. | Fragrance | q.l. |
| 12. | Purified water | balance |

| | | |
|---|---|---|
| 1) Branched polyether-modified silicone; KF6028 from Shin-Etsu Chemical Co., Ltd. 2) Treated powder; obtained by dissolving 3 g of organopolysiloxane of Preparation Example 2 in isopropyl alcohol, adding 97 g of each powder, dispersing the resulting mixture and distilling the solvent off therefrom. | | |

### Preparation Procedures

A: Components 1 to 4 were mixed and component 5 to 7 were added to disperse homogenously.
B: Components 8 to 10 and 12 were dissolved homogeneously.
C: While stirring, B was added to A to emulsify and component 11 was added thereto to obtain eyeshadow.

The cream eyeshadow thus obtained demonstrated a light spreadability without oily look nor powdery look and gave moisture and a refreshing feeling to users. It also had a good water resistance and a sweat resistance and makeup coverage lasted long. No quality change was found with temperature and time.

### Example 25: Lipstick

| Component | | Weight % |
|---|---|---|
| 1. | Candelilla wax | 8.0 |
| 2. | Polyethylene wax | 8.0 |
| 3. | Long-chain alkyl group containing acrylic silicone resin ¹⁾ | 12.0 |
| 4. | Methylphenylpolysiloxane ²⁾ | 3.0 |
| 5. | Isotridecyl isononanate | 20.0 |
| 6. | Glyceryl isostearate | 16.0 |
| 7. | Polyglyceryl triisostearate | 28.5 |
| 8. | Red No. 202 treated with organopolysiloxane from Preparation Example 3 ³⁾ | 0.8 |
| 9. | Iron oxide red treated with organopolysiloxane from Preparation Example 3 ³⁾ | 1.5 |
| 10. | Iron oxide yellow treated with organopolysiloxane from Preparation Example 3 ³⁾ | 1.0 |
| 11. | Iron oxide black treated with organopolysiloxane from Preparation Example 3 ³⁾ | 0.2 |
| 12. | Titanium dioxide treated with organopolysiloxane from Preparation Example 3 ³⁾ | 1.0 |
| 13. | Antiseptics | q.l. |
| 14. | Fragrance | q.l. |

| | | |
|---|---|---|
| 1) Long-chain alkyl group containing acrylic silicone resin; KP-561P from Shin-Etsu Chemical Co., Ltd. 2) Methylphenylpolysiloxane; KF-54 from Shin-Etsu Chemical Co., Ltd. 3) Treated powder; obtained by dissolving 2 g of the organopolysiloxane of Preparation Example 3 in isopropyl alcohol, adding 98g of each powder, dispersing the resulting mixture, and distilling the solvent off therefrom. | | |

### Preparation Procedures

A: Components 1 to 6 were mixed and part of component 6 was mixed to dissolve.
B: Components 8 to 14 and the rest of the component 7 was mixed homogeneously and the resulting mixture was added to A to obtain a homogenous mixture.

The lipstick thus obtained demonstrated a light spreadability without oily look nor powdery look and gave a refreshing feeling to users. It also showed a superior stability with a good water resistance and water repellency.

### Example 26: Eyeliner

| Component | | Weight % |
|---|---|---|
| 1. | Decamethylcyclopentasiloxane | 6.0 |
| 2. | Dimethylpolysiloxane (6 mm²/sec at 25 degree C) | 5.0 |
| 3. | Jojoba oil | 2.0 |
| 4. | Polyether-modified silicone ¹⁾ | 1.0 |
| 5. | Alkyl/polyether co-modified silicone ²⁾ | 1.0 |
| 6. | Acrylic silicone resin ³⁾ | 15.0 |
| 7. | Iron oxide black treated with organopolysiloxane from Preparation Example 4 ⁴⁾ | 20.0 |
| 8. | Ethanol | 5.0 |
| 9. | Antiseptics | q.l. |
| 10. | Purified water | q.l. |

| | | |
|---|---|---|
| 1) Polyether-modified silicone; KF6017 from Shin-Etsu Chemical Co., Ltd. 2) Alkyl/polyether co-modified silicone; KF6026 from Shin-Etsu Chemical Co., Ltd. 3) Acrylic silicone resin; KP545 from Shin-Etsu Chemical Co., Ltd. 4) Iron oxide black treated with organopolysiloxane; obtained by dissolving 2 g of the organopolysiloxane of Preparation Example 4 in isopropyl alcohol, adding 98g of iron oxide black, dispersing the resulting mixture, and distilling the solvent off therefrom. | | |

### Preparation Procedures

A: Components 1 to 6 were mixed while heating and component 7 was added. The resulting mixture was dispersed homogenously.
B: Components 8 to 10 were dissolved while heating
C: While stirring, B was added to A. The resulting mixture was emulsified to obtain eyeliner.

The eyeliner thus obtained demonstrated a light spreadability without oily look nor powdery look and gave moisture and a refreshing feeling to users. It also had a good water resistance and a sweat resistance and makeup coverage lasted long. No quality change was found with temperature and time.

### Example 27: Liquid Foundation

| Component | | Weight % |
|---|---|---|
| 1. | Dimethylpolysiloxane (6 mm²/sec at 25 degree C) | 4.5 |
| 2. | Decamethylcyclopentasiloxane | 15.0 |
| 3. | Squalane | 4.0 |
| 4. | Neopentylglycol dioctanoate | 3.0 |
| 5. | Myristic acid isostearic acid diglyceride | 2.0 |
| 6. | α-Monoisostcaryl glyceryl ether | 1.0 |
| 7. | Polyether-modified silicone 1) | 1.0 |
| 8. | Alkyl/polyether co-modified silicone ²⁾ | 0.5 |
| 9. | Aluminum distearate | 0.2 |
| 10. | Titanium dioxide treated with organopolysiloxane from Preparation Example 2 ³⁾ | 5.0 |
| 11. | Sericite treated with organopolysiloxane from Preparation Example 4 ³⁾ | 2.0 |
| 12. | Talc treated with organopolysiloxane from Preparation Example 4 ³⁾ | 3.0 |
| 13. | Iron oxide red treated with organopolysiloxane from Preparation Example 4 ³⁾ | 0.4 |
| 14. | Iron oxide yellow treated with organopolysiloxane from Preparation Example 4 ³⁾ | 0.7 |
| 15. | Iron oxide black treated with organopolysiloxane from Preparation Example 4 ³⁾ | 0.1 |
| 16. | Magnesium sulfate | 0.7 |
| 17. | Glycerin | 3.0 |
| 18. | Antiseptics | q.l. |
| 19. | Fragrance | q.l. |
| 20. | Purified water | balance |

| | | |
|---|---|---|
| 1) Polyehter-modifeid silicone; KF6019 from Shin-Etsu Chemical Co., Ltd. 2) Alkyl/polyether co-modified silicone; KF6026 from Shin-Etsu Chemical Co., Ltd. 3) Treated powder; obtained by dissolving 2 g of the organopolysiloxane of Preparation Example 4, adding 98 g of powder, dispersing the resulting mixture, and distilling the solvent off therefrom | | |

### Preparation Procedures

A: Components 1 to 9 were mixed while heating and component 10 to 15 were added. The resulting mixture was dispersed homogenously.
B: Components 16 to 18 and compnent 20 were dissolved while heating.
C: While stirring, B was added dropwise to A. The resulting mixture was emulsified, cooled, and component 10 was added to thereby liquid emulsified foundation.

The liquid emulsified foundation thus obtained was found to have a fine texture with non-sticky, non-oily, moisturizing, and hydrating touch and spread lightly. It could also provide cool feeling for skin and makeup coverage lasted long. In addition, it was found that the present liquid emulsified foundation did not cause quality change with temperature and time, having a very excellent stability.

### Example 28: Liquid Emulsified Foundation

| Component | | Weight % |
|---|---|---|
| 1. | Decamethylcyclopentasiloxane | 16.0 |
| 2. | Dimethylpolysiloxane (6 mm²/sec at 25 degree C) | 8.0 |
| 3. | Octyl paramethoxycinnamate | 3.0 |
| 4. | 12-Hydroxystearic acid | 1.0 |
| 5. | Fluorine-modified silicone ¹⁾ | 15.0 |
| 6. | Fluorinated alkyl/polyether co-modified silicone ²⁾ | 5.0 |
| 7. | Powder of spherical polymethylsilsesquioxane ³⁾ | 3.0 |
| 8. | Fine powder of titanium dioxide treated with organopolysiloxane from Preparation | |
| | Example 3⁴⁾ | 8.0 |
| 9. | Mica titanium dioxide treated with organopolysiloxane from Preparation | Example 3 ⁴⁾ |
| | | 1.0 |
| 10. | Titanium dioxide treated with organopolysiloxane from Preparation | Example 3 ⁴⁾ |
| | | 5.0 |
| 11. | Iron oxide red treated with organopolysiloxane from Preparation Example | 3 ⁴⁾ |
| | | 0.9 |
| 12. | Iron oxide yellow treated with organopolysiloxane from Preparation | Example 3 ⁴⁾ |
| | | 2.0 |
| 13. | Iron oxide black treated with organopolysiloxane from Preparation Example | 3 ⁴⁾ |
| | | 1.0 |
| 14. | Ethanol | 15.0 |
| 15. | Glycerin | 3.0 |
| 16. | Magnesium sulfate | 1.0 |
| 17. | Antiseptics | q.l. |
| | 18. Fragrance | q.l. |
| | 19. Purified water | balance |

| | | |
|---|---|---|
| 1) Fluorine-modified silicone; FL-50 from Shin-Etsu Chemical Co.,Ltd. 2) Flouorinated alkyl/polyether-comodified silicone; FPD-4694 from Shin-Etsu Chemical Co., Ltd. 3) Powder of spherical polymethylsilsesquioxane; KMP 590 50 from Shin-Etsu Chemical Co.,Ltd. 4) Treated powder; obtained by dissolving 2 g of the organopolysiloxane of Preparation Example 3, adding 98g of each powder, dispersing the resulting mixture, and distilling the solvent off therefrom. | | |

### Preparation Procedures

A: Components 7 to 13 were mixed homogenously.
B: Components 1 to 6 were mixed while heating to 70 degree C and A was added. The resulting mixture was dispersed homogeneously.
C: The mixture of components 14 to 17 and components 19 was heated to 40 degree C, the mixture was added dropwise to B. The resulting mixture was emulsified, cooled, and component 18 was added to obtain liquid foundation.

The liquid emulsified foundation thus obtained demonstrated a light spreadability without stickiness and gave a non-oily and refreshing feeling to users. It was also found that the present liquid emulsified foundation did not cause quality change with temperature and time, having a very excellent stability.

### Example 29: Eyeliner

| Component | | Weight % |
|---|---|---|
| 1. | Decamethylcyclopentasiloxane | 22.0 |
| 2. | Dimethylpolysiloxane (6 mm²/sec at 25 degree C) | 5.0 |
| 3. | Iron oxide black treated with organopolysiloxane from Preparation Example 1 ¹⁾ | 20.0 |
| 4. | Organosilicone resin ²⁾ | 10.0 |
| 5. | Vitamin E acetate | 0.2 |
| 6. | Jojoba oil | 2.0 |
| 7. | Bentonite | 3.0 |
| 8. | Polyether-modified silicone ³⁾ | 2.0 |
| 9. | Ethanol | 3.0 |
| 10. | 1,3-Butylene glycol | 5.0 |
| 11. | Antiseptics | q.l. |
| 12. | Purified water | q.l. |

| | | |
|---|---|---|
| 1) Iron oxide black treated with organopolysiloxane: obtained by dissolving 2 g of the organopolysiloxane of Preparation Example 1, adding 98g of iron oxide black, dispersing the resulting mixture, and distilling the solvent off therefrom. 2) Organosilicone resin; KF-7312J from Shin-Etsu Chemical Co., Ltd. 3) Polyether-modified silicone; KF6017 from Shin-Etsu Chemical Co., Ltd. | | |

### Preparation Procedures

A: Components 1, 2, and from 4 to 8 were mixed and thereto component 3 was added. The resulting mixture was dispersed homogeneously.
B: Components 9 to 11 and 13 were mixed.
C: B was added dropwise to A. The resulting mixture was emulsified and cooled to obtain eyeliner.

The eyeliner thus obtained demonstrated a light spreadability and was easy to draw with. It also gave a refreshing and non oily feelings to users. No quality change was found with temperature and time and it showed superior stability and good users' satisfaction. It was also found that cosmetic effect maintained long with excellent water resistance and sweat resistance.

### Example 30: Foundation

| Component | | Weight % |
|---|---|---|
| 1. | Decamethylcyclopentasiloxane | 27.0 |
| 2. | Methylphenylpolysiloxane | 3.0 |
| 3. | Glyceryl trioctanoate | 10.0 |
| 4. | Branched polyglycerin modified silicone ¹⁾ | 1.0 |
| 5. | Polyglyceryl monoisostearate | 3.0 |
| 6. | Mixture of hydrophobized powders ²⁾ | 18.0 |
| 7. | Iron oxie red | 1.2 |
| 8. | Iron oxide yellow | 2.6 |
| 9. | Iron oxide black | 0.2 |
| 10. | 1,3-Butylene glycol | 7.0 |
| 11. | Sodium chloride | 0.5 |
| 12. | Antiseptics | q.l. |
| 13. | Fragrance | q.l. |
| 14. | Purified water | balance |

| | | |
|---|---|---|
| 1) Branched polyglycerin-modified silicone; KF6104 from Shin-Etsu Chemical Co., Ltd. 2) Mixture of hydrophobized powders; a.Fine powder of titanium 8.0 b. Fine powder of zinc oxide 4.0 c. Talc 3.0 d. Mica 3.0 | | |

### Preparation Procedures

A: Components from a to d were mixed. To the resulting powder mixture was added 1 wt.% of organopolysiloxane from Preparation Example 2 to heat.
B. Components 1 to 5 were mixed and the resulting mixture was dissolved while heating. Components 6 to 9 were added and then the mixture was dispersed homogeneously.
C. Components 10 to 12 and 14 were mixed. The resulting mixture was added to B and the resulting mixture was emulsified.
D: C was cooled and thereto component 13 was added to obtain foundation.

The foundation thus obtained was non-sticky, had a light spreadability, a good adhesion, and a shiny gloss, and well fit toward skin. Makeup coverage maintained long, and it was also found that the present foundation did not cause quality change with temperature and time, having a very excellent stability.

### Example 31: Hair Spray for Brushing

| Component | | Weight % |
|---|---|---|
| 1. | Isopropyl myristate | 1.0 |
| 2. | Stearyltrimethylammonium chloride | 0.05 |
| 3. | Zinc oxide composition of Example 6 (H) | 3.0 |
| | 4. Ethanol | 25.0 |
| | 5. Fragrance | q.l. |
| 6. | Blowing agent | balance |

### Preparation Procedures

A: Components 1 to 5 were mixed.
B: A was packed into an aerosol can and then component 6 was packed to obtain brushing agent.

Brushing spray thus obtained was found to give a shiny and very smooth finish, of which effect maintained long. It also demonstrated a good dispersibility of powder when using and left hair easy to comb.

### Example 32: Rinse

| Component | | Weight % |
|---|---|---|
| 1. | Ethylene glycol distearate | 3.0 |
| 2. | Cetanol | 2.0 |
| 3. | Propylene glycol monostearate | 3.0 |
| 4. | Dimethylpolysiloxane (100 mm²/sec at 25 degree C) | 3.0 |
| 5. | Glycerin monostearate | 4.0 |
| 6. | Polyoxyethylene (3) stearate | 4.0 |
| 7. | Acetyltrimethylammonium chloride | 5.0 |
| 8. | Polyoxyethylene (20) cetyl ether | 2.0 |
| 9. | Zinc oxide composition of Example 6 (H) | 2.0 |
| 10. | 1,3-Butylene glycol | 5.0 |
| 11. | Antiseptics | q.l. |
| 12. | Fragrance | q.l. |
| 13. | Purified water | balance |

### Preparation Procedures

A: Components 1 to 9 were combined and the resulting mixture was stirred to mix.
B. Components 10,11 and 13 were mixed while heating.
C: B was added to A to mix and then the resulting mixture was cooled and component 12 was added to obtain rinse.

Rinse thus obtained was found to be non-sticky, leave hair no heavy feeling upon using but a shiny gloss and a light and smooth touch, and add volume to hair. It also left hair easy to comb, and the effect maintained long and users satisfaction was good.

### Example 33: No Rinse Shampoo

| Component | | Weight % |
|---|---|---|
| 1. | Lauric acid amide propyldimethylaminoacetic acid betaine (30%) | 15.0 |
| 2. | Sodium polyoxyethylene (3) lauryl ether sulfate (27%) | 4.0 |
| 3. | Polyoxyethylene (150) distearate | 0.5 |
| 4. | Cationized cellulose (4%) | 0.5 |
| 5. | Glycerin | 3.0 |
| 6. | Dimethylpolysiloxane (1000,000 mm²/sec at 25 degree C) | 1.0 |
| 7. | Dimethylpolysiloxane (100 mm²/sec at 25 degree C) | 3.0 |
| 8. | Mica treated with organopolysiloxane from Preparation Example 3 | 2.0 |
| 9. | Antiseptics | q.l. |
| 10. | Fragrance | q.l. |
| 11. | Purified water | balance |

| | | |
|---|---|---|
| 1) Mica treated with organopolysiloxane; obtained by dissolving 2 g of the organopolysiloxane of Preparation Example 3 in isopropyl alcohol, adding 98g of mica, dispersing the resulting mixture and distilling the solvent off therefrom. | | |

### Preparation Procedures

A: Components 1 to 5,9 and 11 were combined and mixed while heating.
B. Components 6 to 8 were mixed and the resulting mixture was dispersed.
C: B was added to A to mix and then, the resulting mixture was cooled. Component 10 was added to obtain no rinse shampoo.

No rinse shampoo thus obtained was found to be non-sticky, leave hair no heavy feeling upon using but a shiny gloss and a light and smooth touch, and add volume to hair. It also left hair easy to comb, and the effect maintained long and users satisfaction was good.

### Example 34: Treatment

| Component | | Weight % |
|---|---|---|
| 1. | Ethylene glycol distearate | 1.0 |
| 2. | Liquid paraffins | 10.0 |
| 3. | Squalane | 5.0 |
| 4. | Stearyl alcohol | 1.5 |
| 5. | Dimethylpolysiloxane (10 mm²/sec at 25 degree C) | 3.0 |
| 6. | Stearic acid | 6.0 |
| 7. | Polyoxyethylene (3) stearyl alcohol | 4.5 |
| 8. | Polyoxyethylene (150) cetyl alcohol | 2.0 |
| 9. | Sericite treated with organopolysiloxane from Preparation Example 2 ¹⁾ | 1.5 |
| 10. | 1,3-Butylene glycol | 6.0 |
| 11. | Antiseptics | q.l. |
| 12. | Fragrance | q.l. |
| 13. | Purified water | balance |

| | | |
|---|---|---|
| 1) Sericite treated with organopolysiloxane from Preparation Example 2; obtained by dissolving 2 g of the organopolysiloxane of Preparation Example 2 in isopropyl alcohol, adding 98g of sericite, dispersing the resulting mixture, and distilling the solvent off therefrom. | | |

### Preparation Procedures

A: Components 1 to 9 were mixed while heating.
B. Components 10, 11, and 13 were mixed and the resulting mixture was dispersed.
C: B was added to A to mix thereof, and then the resulting mixture was cooled. Component 10 was added to obtain treatment.

Treatment thus obtained was found to be non-sticky, leave hair no heavy feeling upon using but a shiny gloss and a light and smooth touch, and add volume to hair. It also left hair easy to comb, and the effect maintained long and users satisfaction was good.

### Example 35: Water-in-oil Type Antiperspirant

| Component | | Weight % |
|---|---|---|
| 1. | Crosslinked polyether-modified silicone ¹⁾ | 7.0 |
| 2. | Decamethylcyclopentasiloxane | 10.0 |
| 3. | Glyceryl trioctanoate | 7.0 |
| 4. | Dipropylene glycol | 5.0 |
| 5. | Sodium citrate | 0.2 |
| 6. | Aluminum zirconium tetrachlorohydrate | 18.0 |
| 7. | Zinc oxide composition of Example 7 (I) | 5.0 |
| 8. | Composite powder of fluorine-modified hybrid silicone ²⁾ | 2.0 |
| 9. | Fragrance | q.l. |
| 10. | Purified water | 45.8 |

| | | |
|---|---|---|
| 1) Crosslinked polyether-modified silicone; KSG-210 from Shin-Etsu Chemical Co., Ltd. 2) Composite powder of fluorine-modified hybrid silicone; KSP-200 from Shin-Etsu Chemical Co., Ltd. | | |

### Preparation Procedures

A: Components 1 to 3 were mixed.
B. Components 4 to 10 were mixed.
C: B was added to A and the resulting mixture was mixed to emulsify.

Water-in-oil type antiperspirant thus obtained was found to spread lightly and leave hair non-stiky nor oily touch but a cool and refreshing feeling. In addition, it was found that the present antiperspirant caused no change in quality with temperature and time, having good users satisfaction and a good stability.

### Example 36: Antiperspirant of Roll-on-Type

| Component | | Weight % |
|---|---|---|
| 1. | Crosslinked polyether-modified silicone ¹⁾ | 20.0 |
| 2. | Dimethylpolysiloxane (6 mm²/sec at 25 degree C) | 10.0 |
| 3. | Crosslinked dimethylpolysiloxane ²⁾ | 15.0 |
| 4. | Decamethylcyclopentasiloxane | 30.0 |
| 5. | Aluminum/zirconium tetrachlorohydrate | 20.0 |
| 6. | Zinc oxide composition of Example 6 (H) | 4.0 |
| 7. | Composite powder of phenyl-modified hybrid silicone ³⁾ | 1.0 |
| 8. | Fragrance | q.l. |

| | | |
|---|---|---|
| 1) Crosslinked polyether-modified silicone; KSG-210 from Shin-Etsu Chemical Co., Ltd. 2) Crosslinked dimethylpolysiloxane, KSG-15 from Shin-Etsu Chemical Co., Ltd. 3) Composite powder of Phenyl-modified hybrid silicone; KSP-300 from Shin-Etsu Chemical Co., Ltd. | | |

### Preparation Procedures

A: Components 1 to 4 were mixed.
B. Components 5 to 8 were combined to A and the resulting mixture was dispersed homogeneously.

Antiperspirant of roll-on-type thus obtained was found to spread lightly and have non-sticky or non-oily touch and leave skin a cool and refreshing feeling. In addition, it was found that the present antiperspirant of roll-on-type caused no change in quality with temperature and time, having good users satisfaction and a good stability.

### Example 37: Suncut Milky Lotion

| Component | | Weight % |
|---|---|---|
| 1. | Decamethylcyclopentasiloxane | 8.0 |
| 2. | Methylphenylpolysiloxane | 3.0 |
| 3. | Sorbitan monoisostearate | 1.0 |
| 4. | Polyether modified silicone ¹⁾ | 0.5 |
| 5. | Trimethylsiloxy cinnamate ²⁾ | 1.0 |
| 6. | Octyl paramethoxy cinnamate | 4.0 |
| 7. | Titanium dioxide dispersion of Example 1 (A) | 20.0 |
| 8. | Sorbitol | 2.0 |
| 9. | Sodium chloride | 2.0 |
| 10. | Antiseptics | q.l. |
| 11. | Fragrance | q.l. |
| 12. | Purified water | balance |

| | | |
|---|---|---|
| 1) Polyether-modified silicone; KF-6015 from Shin-Etsu Chemical Co., Ltd. 2) Trimethylsiloxy cinnamate; X-21-5250 from Shin-Etsu Chemical Co., Ltd. | | |

### Preparation Procedures

A: Components 1 to 6 were mixed while heating and component 7 was dispersed homogeneously therein.
B. Components 8 to 10, and 12 were mixed while heating.
C: While stirring, B was added dropwise to A. The resulting mixture was cooled and component 11 was added to obtain suncut milky lotion.

Suncut milky lotion thus obtained was found to have a fine texture with a light spreadability and have a non-sticky touch and leave skin moisturized and hydrated feeling as well as a long lasting coverage effect. Ultraviolet ray protection effect maintained long. In addition, it was found that the present suncut milky lotion caused no change in quality with temperature changes and time, having a very excellent stability.

### Example 38: Suncut Milky Lotion

| Component | | Weight % |
|---|---|---|
| 1. | Dimethylpolysiloxane (6 mm²/sec at 25 degree C) | 3.0 |
| 2. | Decamethylcyclopentasiloxane | 3.0 |
| 3. | Glyceryl trioctanoate | 2.0 |
| 4. | Crosslinked polyether-modified silicone ¹⁾ | 3.0 |
| 5. | Crosslinked dimethylpolysiloxane ²⁾ | 2.0 |
| 6. | Branched polyether co-modified silicone ³⁾ | 1.0 |
| 7. | Titanium dioxide dispersion of Example 1 (A) | 25.0 |
| 8. | Zinc oxide dispersion of Example 2 (B) | 35.0 |
| 9. | Sodium citrate | 0.2 |
| 10. | Dipropylene glycol | 3.0 |
| 11. | Antiseptics | q.l. |
| 12. | Fragrance | q.l. |
| 13. | Purified water | balance |

| | | |
|---|---|---|
| 1) Crosslinked polyether-modified silicone; KSG210 from Shin-Etsu Chemical Co., Ltd. 2) Crosslinked dimethylpolysiloxane; KSG15 from Shin-Etsu Chemical Co., Ltd. 3) Branched polyether-co-modified silicone; KF6028 from Shin-Etsu Chemical Co., Ltd. | | |

### Preparation Procedures

A: Components 1 to 6 were combined to mix homogenously.
B: Components 9 to 11 and 13 were mixed and dissolved.
C: B was added to A to emulsify and components 8, 9 and 12 were added to obtain suncut milky lotion.

The suncut milky lotion thus obtained was non-sticky, had a light spreadability, a good adhesion, provided a shiny finish, and well fit toward skin. Makeup coverage maintained long, and it was also found that the present suncut milky lotion did not cause quality change with temperature and time, having a very excellent stability.

### Example 39: Suncut Cream

| Components | | Weight % |
|---|---|---|
| 1. | Decamethylcyclopentasiloxane | 17.5 |
| 2. | Acrylic silicone resin ¹⁾ | 12.0 |
| 3. | Glyceryl trioctanoate | 5.0 |
| 4. | Octyl paramethoxy cinnamate | 6.0 |
| 5. | Crosslinked polyether-modified silicone ²⁾ | 5.0 |
| 6. | Alkyl / polyether co modified silicone ³⁾ | 1.0 |
| 7. | Zinc oxide composition of Example 6 (H) | 20.0 |
| 8. | Sodium chloride | 0.5 |
| 9. | 1,3-Butylene glycol | 2.0 |
| 10. | Antiseptics | q.l. |
| 11. | Fragrance | q.l. |
| 12. | Purified water | balance |

| | | |
|---|---|---|
| 1) Acrylic silicone resin; KP545 from Shin-Etsu Chemical Co., Ltd. 2) Crosslinked polyether-modified siicone; KSG210 from Shin-Etsu Chemical Co., Ltd. 3) Alkyl / polyether co-modified silicone; KF6026 from Shin-Etsu Chemical Co., Ltd. | | |

### Preparation Procedures

A: Component 2 was added to a part of component 1 to mix homogeneously, and thereto component 7 was added and the resulting mixture was dispersed with the use of beads mill.
B: The rest of the component 1 and components 3 to 6 were combined to mix homogeneously.
C: Components 8 to 10 and 12 were combined to dissolve.
D: C was added to B and the resulting mixture was emulsified. A and component 11 were added to obtain sunscut cream.

The suncut cream thus obtained was non-sticky, had a light spreadability, a good adhesion, provided a shiny finish, and well fit toward skin. Makeup coverage maintained long, and it was also found that the present suncut cream did not cause quality change with temperature and time, having a very excellent stability.

### Example 40: O / W Type Suncut Milky Lotion

| Components | | Weight % |
|---|---|---|
| 1. | Acrylic silicone resin¹⁾ | 10.0 |
| 2. | Titanium dioxide dispersion of Example 1 | 15.0 |
| 3. | Zinc oxide dispersion of Example 2 | 15.0 |
| 4. | Decamethylcyclopentasiloxane | 16.5 |
| 5. | Neopentylglycol dioctanoate | 8.0 |
| 6. | Polymethylsesquioxane | 3.0 |
| 7. | Polyether-modified silicone ²⁾ | 4.0 |
| 8. | Polyether-modified silicone ³⁾ | 1.5 |
| 9. | Polyoxyethylene hydrogenated castor oil | 1.5 |
| 10. | 1,3-Butylene glycol | 5.0 |
| 11. | Purified water | 20.5 |

| | | |
|---|---|---|
| 1) Acrylic silicone resin KP-545 from Shin-Etsu Chemical Co., Ltd. 2) Polyether-modified silicone; KF-6018 from Shin-Etsu Chemical Co., Ltd. 3) Polyether-modified silicone; KF-6029 from Shin-Etsu Chemical Co., Ltd. | | |

### Preparation Procedures

A: Components 7 to 11 were mixed homogeneously.
B: Components 1 to 6 were combined to disperse homogeneously and the resulting dispersion was added to A to obtain suncut milky lotion.

Suncut milky lotion thus obtained was found to have a fine texture with a light spreadability and have a non-sticky touch and leave skin moisturized and hydrated feeling as well as a long lasting coverage effect. Ultraviolet ray protection effect maintained long. In addition, it was found that the present suncut milky lotion caused no change in quality with temperature changes and time, having a very excellent stability.

### EFFECT OF THE INVENTION

The powder composition comprising siloxane compound according to the general formula (1) and the dispersion of powder in oil comprising siloxane compound according to the general formula (1) have an excellent dispersibitlity, do not cause aggregation nor settling of powder with time, and do not arise odor. The cosmetics according to the present invention formulating the powder and the dispersion of the powder in oil have a light spreadability, non-oily touch, leave skin moisturized and hydrated, and provide refreshed feelings upon using. In addition, makeup coverage maintains long and it was also found that the present cosmetics did not cause quality change with temperature and time, having a very excellent stability.

## Claims

1. A dispersion of powder in oil, consisting of the polyglycerin modified silicone having at least one silicone branch, powder and/or a coloring agent, and an oily medium, wherein the polyglycerin modified silicone is represented by the following formula (1)
R¹ₐR²_{b}R³_{c}SiO_{(4-a-b-c)/2} (1)
wherein R¹ may be same with or different from each other and is an organic radical selected from the group consisting of C₁₋₃₀ alkyl radicals, aryl radicals, aralkyl radicals, fluorinated alkyl radicals, amino-substituted alkyl radicals, carboxyl substituted alkyl radicals, or organic radicals having the general formula (2),
-C_{d}H_{2d}-O-(C₂H₄O)ₑ(C₃H₆O)_{f}R⁴ (2)
wherein 0≤d≤15, 0≤e≤50, and 0≤f≤50,
R² is a polyglycerin derivative having the general formula (3) or (4), 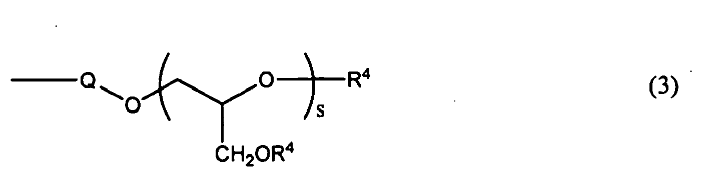 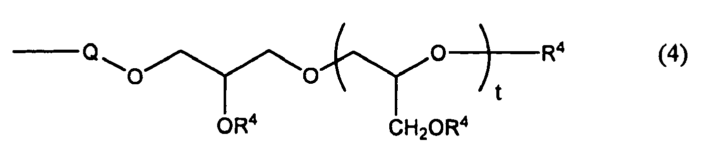 wherein R⁴ is a hydrogen atom, a C₁₋₃₀ alkyl, or R⁵-(CO)-, R⁵ being a C_{1 - 30} hydrocarbyl radical, and Q is a divalent C₃₋₂₀ hydrocarbyl radical which may contain an ether or ester bond, s is an integer of 2 to 20, and t is an integer of 1 to 20, and
R³ is organosiloxane having the general formula (5), 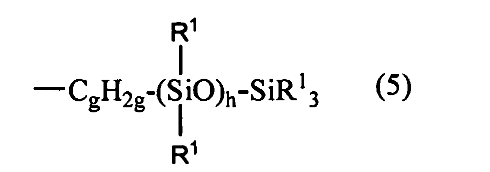 wherein g is an integer of 1 ≤ g ≤ 50 and h is an integer of 0 ≤ h ≤ 500 and
1.0 ≤ a ≤ 2.5, 0.001 ≤ b ≤ 1.5, and 0.001 ≤ c ≤ 1.5.

2. The dispersion of powder in oil according to claim 1, wherein said polyglycerin-modified silicone is contained in an amount of 0.1 to 30 parts by weight per 100 parts by weight of said powder and said coloring agent.

3. The dispersion of powder in oil according to claim 1 or 2, wherein the powder is zinc oxide.

4. The dispersion of powder in oil according to claim 1 or 2, wherein the powder is titanium dioxide.

5. The dispersion of powder in oil according to claim 1 or 2, wherein the powder is an extender pigment such as mica, sericite, talc, and kaolin.

6. The dispersion of powder in oil according to any one of claims 1 to 5, wherein the oily medium is an organopolysiloxane selected from the group consisting of dimethylpolysiloxane, methylphenylpolysiloxane, octamethylcyclotetrasiloxane, decamethylcyclopentasiloxane and dodecamethylcyclohexasiloxane.

7. Use of the dispersion of powder in oil according to any one of claims 1 to 6, wherein the dispersion of powder in oil is a raw material for preparing a cosmetic.

## Patentansprüche

1. Dispersion eines Pulvers in Öl, bestehend aus dem Polyglycerin-modifizierten Silikon mit mindestens einer Silikonverzweigung, Pulver und/oder einem färbenden Mittel und einem öligen Medium, wobei das Polyglycerin-modifizierte Silikon durch die folgende Formel (1) dargestellt wird
R¹ₐR²_{b}R³_{c}SiO_{(4-a-b-c)/2} (1)
worin die R¹ gleich oder verschieden voneinander sein können und R¹ ein organischer Rest ist, ausgewählt aus der Gruppe, bestehend aus C₁₋₃₀-Alkylresten, Arylresten, Aralkylresten, fluorierten Alkylresten, aminosubstituierten Alkylresten, carboxylsubstituierten Alkylresten oder organischen Resten mit der allgemeinen Formel (2)
-C_{d}H_{2d}-O-(C₂H₄O)ₑ(C₃H₆O)_{f}R⁴ (2)
worin 0 ≤ d ≤ 15, 0 ≤ e ≤ 50 und 0 ≤ f ≤ 50,
R² ein Polyglycerinderivat mit der allgemeinen Formel (3) oder (4) ist 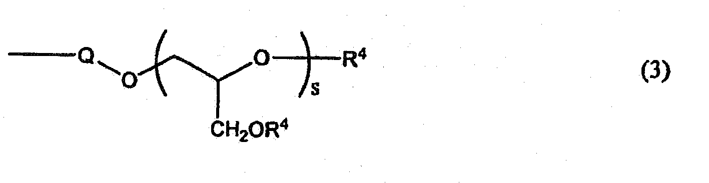 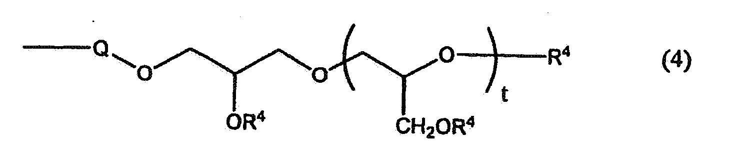 worin R⁴ ein Wasserstoffatom, ein C₁₋₃₀-Alkyl oder R⁵-(CO)-ist, wobei R⁵ ein C₁₋₃₀-Kohlenwasserstoffrest ist und Q ein divalenter C₃₋₂₀-Kohlenwasserstoffrest ist, welcher eine Ether- oder eine Esterbindung enthalten kann, s eine ganze Zahl von 2 bis 20 ist und t eine ganze Zahl von 1 bis 20 ist, und
R³ ein Organosiloxan mit der allgemeinen Formel (5) ist 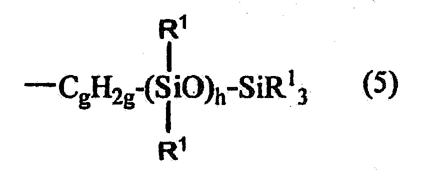 worin g eine ganze Zahl von 1 ≤ g ≤ 50 ist und h eine ganze Zahl von 0 ≤ h ≤ 500 ist und 1,0 ≤ a ≤ 2,5, 0,001 ≤ b ≤ 1,5 und 0,001 ≤ c ≤ 1,5.

2. Dispersion eines Pulvers in Öl gemäß Anspruch 1, wobei das Polyglycerin-modifizierte Silikon in einer Menge von 0,1 bis 30 Gewichtsteilen pro 100 Gewichtsteilen des Pulvers und des färbenden Mittels enthalten ist.

3. Dispersion eines Pulvers in Öl gemäß Anspruch 1 oder 2, wobei das Pulver Zinkoxid ist.

4. Dispersion eines Pulvers in Öl gemäß Anspruch 1 oder 2, wobei das Pulver Titandioxid ist.

5. Dispersion eines Pulvers in Öl gemäß Anspruch 1 oder 2, wobei das Pulver ein Streckmittelpigment, wie Glimmer, Sericit, Talkum und Kaolin, ist.

6. Dispersion eines Pulvers in Öl gemäß einem der Ansprüche 1 bis 5, wobei das ölige Medium ein Organopolysiloxan ist, ausgewählt aus der Gruppe, bestehend aus Dimethylpolysiloxan, Methylphenylpolysiloxan, Octamethylcyclotetrasiloxan, Decamethylcyclopentasiloxan und Dodecamethylcyclohexasiloxan.

7. Verwendung der Dispersion eines Pulvers in Öl gemäß einem der Ansprüche 1 bis 6, wobei die Dispersion eines Pulvers in Öl ein Rohmaterial zur Herstellung eines Kosmetikums ist.

## Revendications

1. Dispersion de poudre dans de l'huile constituée du silicone polyglycérine-modifié présentant au moins une ramification silicone, d'une poudre et/ou d'un agent colorant, et d'un milieu huileux, dans laquelle le silicone polyglycérine-modifié est représenté par la formule (1) suivante
R¹ₐR²_{b}R³_{c}SiO_{(4-a-b-c)/2} (1)
dans laquelle R¹ peuvent être identiques ou différents les uns des autres et sont un radical organique choisi parmi des radicaux alkyle, des radicaux aryle, des radicaux aralkyle, des radicaux alkyle fluorés, des radicaux alkyle amino-substitués, des radicaux alkyle carboxyle-substitués en C₁-C₃₀ ou des radicaux organiques présentant la formule (2) générale :
-C_{d}H_{2d}-O-(C₂H₄O)ₑ(C₃H₆O)_{f}R⁴ (2)
dans laquelle 0 ≤ d ≤ 15,0 ≤ e ≤ 50, et 0 ≤ f ≤ 50,
R² est un dérivé de polyglycérine présentant la formule générale (3) ou (4), 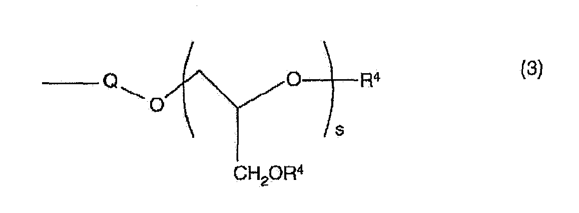 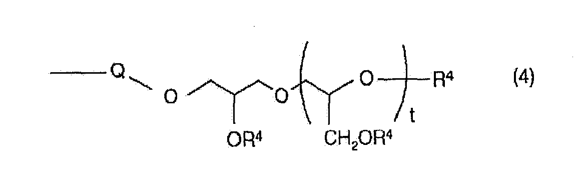 dans lesquelles R⁴ est un atome d'hydrogène, un groupe alkyle en C₁-C₃₀, ou R⁵-(CO)-, R⁵ étant un radical hydrocarbyle en C₁-C₃₀, et Q est un radical hydrocarbyle en C₃-C₂₀ divalent qui peut contenir une liaison éther
ou ester, s est un nombre entier de 2 à 20 et t est un nombre entier de 1 à 20, et
R³ est un organosiloxane ayant la formule générale (5): 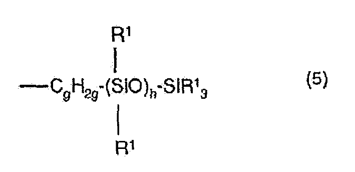 dans laquelle g est un nombre entier de 1 ≤ g < 50 et h est un nombre entier de 0 ≤ h ≤ 500 et 1,0 ≤ a ≤ 2,5, 0,001 ≤ b ≤ 1,5 et 0,001 ≤ c ≤ 1,5.

2. Dispersion de poudre dans de l'huile selon la revendication 1, dans laquelle ledit silicone polyglycérine-modifié est contenu dans une quantité de 0,1 à 30 parties en poids pour 100 parties en poids de ladite poudre et dudit agent colorant.

3. Dispersion de poudre dans de l'huile selon la revendication 1 ou 2, dans laquelle la poudre est de l'oxyde de zinc.

4. Dispersion de poudre dans de l'huile selon la revendication 1 ou 2, dans laquelle la poudre est du dioxyde de titane.

5. Dispersion de poudre dans de l'huile selon la revendication 1 ou 2, dans laquelle la poudre est un pigment diluant, tel que le mica, la séricite, la stéatite et le kaolin.

6. Dispersion de poudre dans de l'huile selon l'une quelconque des revendications 1 à 5, dans laquelle le milieu huileux est un organopolysiloxane choisi parmi le diméthylpolysiloxane, le méthylphénylsiloxane, l'octaméthylcyclotétrasiloxane, le décaméthylcyclopentasiloxane et le dodécaméthylcyclohexasiloxane.

7. Utilisation de la dispersion de poudre dans de l'huile selon l'une quelconque des revendications 1 à 6, dans laquelle la dispersion de poudre dans de l'huile est une matière première pour préparer un cosmétique.
